# EUROPEAN PATENT SPECIFICATION

(11) **EP 2 248 868 B1**
(45) Date of publication and mention of the grant of the patent: **19.09.2018**
(21) Application number: 09714824.1
(22) Date of filing: 20.02.2009
(51) Int. Cl.: C09K 11/06, C07C 211/61, H01L 51/50, H01L 51/00

(54) **LUMINESCENT ELEMENT MATERIAL AND LUMINESCENT ELEMENT**
LEUCHTSTOFFMATERIAL UND LEUCHSTOFFELEMENT
MATÉRIAU POUR ÉLÉMENT LUMINESCENT ET ÉLÉMENT LUMINESCENT

(30) Priority: 27.02.2008 JP 2008045622
(43) Date of publication of application: 10.11.2010
(73) Proprietor: Toray Industries, Inc., Tokyo, 103-8666 (JP)
(72) Inventor: TANAKA, Daisaku, Otsu-shi Shiga 520-8558 (JP); TOMINAGA, Tsuyoshi, Otsu-shi Shiga 520-8558 (JP)
(74) Representative: Hoefer & Partner Patentanwälte mbB
(86) International application number: PCT/JP2009/052961
(87) International publication number: WO 2009/107549

(56) References cited:
- EP-A1- 1 737 277
- WO-A1-00/27946
- JP-A- 2001 326 079
- JP-A- 2002 237 384
- JP-A- 2004 182 737
- US-A1- 2004 106 004
- US-A1- 2005 064 233
- US-A1- 2007 236 137

## Description

### Background of the Invention

### 1. Field of the Invention

The present invention relates to a light emitting device capable of converting electrical energy to light, which can be used for the fields of display devices, flat panel displays, backlights, illumination, interior decoration, signs, signboards, electron cameras and optical signal generators.

### 2. Description of the Related Art

In recent years, studies on an organic thin-film light emitting device which emits light when an electron injected from a cathode and a hole injected from an anode are recombined within an organic emitting materials sandwiched between the electrodes have been actively made. This device is characterized by low-profile, emission of high luminance at a low driving voltage and multicolor emission by selection of an emissive material, and has been attracting attention.

The studies on this device have been made in many research laboratories since C. W. Tang et al. of Kodak have shown that the organic thin-film light emitting device emits light at high luminance. A typical constitution of the organic thin-film light emitting device proposed by a research group of Kodak is a device in which a diamine compound having a hole transporting property, tris(8-quinolinolato)aluminum (III) of an emissive layer, and Mg:Ag as a cathode are sequentially provided on an ITO glass substrate and the device can emit green emission of 1000 cd/m² at a driving voltage of about 10 V (see Applied Physics Letters, (US), 1987, vol. 51, No. 12, 913-915).

The durability of the device is one of the largest problems of the organic thin-film light emitting devices. Particularly with respect to a blue color, there are fewblue emissive materials which can provide a device having excellent durability and high reliability. For example, a technique of using styrylamine derivatives as a blue dopant material (see Japanese Unexamined Patent Publication No. 5-17765 and International Publication WO 2002/020459), and a technique of using-pyrene derivatives as a blue dopant material (see International Publication WO 2004/083162) have been disclosed, but none of these techniques provide a sufficient durability life and color purity. Further, a technique of using chrysene derivatives (see International Publication WO 2004/044088 and Japanese Unexamined Patent Publication No. 2007-230960) has also been disclosed, but the durability life is insufficient though the color purity is high.

A hole injection material for smoothly injecting a hole from an anode into an organic thin-film has also been studied as a technique for improving the durability life by reducing the driving voltage of the organic thin-film light emitting device. As the hole injection materials, copper phthalocyanine compounds (see "Organic EL Material and Display" , CMC Publishing CO., LTD., 2001, p. 151), phenylenediamine derivatives (see Japanese Unexamined Patent Publication No. 2000-309566), and starburstarylamine compounds (see "Organic EL Material and Display", CMC Publishing CO., LTD., 2001, p. 120 and Japanese Unexamined Patent Publication No. 8-291115) have been generally used, but none of them has sufficient durability life.

On the other hand, a technique of using a material having a dibenzochrysene skeleton for an emissive material or a hole transporting material of the organic thin-film light emitting device (see Japanese Unexamined Patent Publication No. 2004-182737 and Proceedings I of 83rd Spring Meeting of the Chemical Society of Japan, B4-40) has also been disclosed.

However, the durability life is not necessarily adequate even when materials described in Japanese Unexamined Patent Publication No. 2004-182737 and Proceedings I of 83rd Spring Meeting of the Chemical Society of Japan, B4-40 are used as a hole injection material. Further, these materials are not used as a blue dopant material and their performance is unknown.

US 2007/236137 A discloses aromatic amine derivatives having a specific structure; and organic electroluminescent devices comprising a cathode, an anode and one or plural organic thin film layers including at least a light emitting layer which are sandwiched between the cathode and the anode wherein at least one of the organic thin film layers contains the above aromatic amine derivatives in the form of a single substance or a component of a mixture. There are provided the organic electroluminescent devices exhibiting a long life and a high efficiency of light emission which are capable of emitting a blue light having high color purity, as well as the aromatic amine derivatives capable of realizing such organic electroluminescent devices.

As described above, there are very few blue dopant materials and hole injection materials which adequately improve the durability life. Accordingly, there is a problem that it is extremely difficult to obtain a light emitting device which is superior in all of luminance efficiency, color purity and the durability life.

### Summary of the Invention

Therefore, it is an object of the present invention to provide a light emitting device material capable of providing a light emitting device which can solve such problems and has high luminance efficiency, excellent color purity and a long life, and a light emitting device using the same.

The present invention is defined in the appended claims and pertains to a light emitting device material used for a light emitting device, which includes at least an emissive layer having a host and a dopant and is present between an anode and a cathode and emits light by electrical energy, wherein the light emitting device material is a light emitting device material for a dopant, having a dibenzochrysene skeleton represented by the following general formula (1): 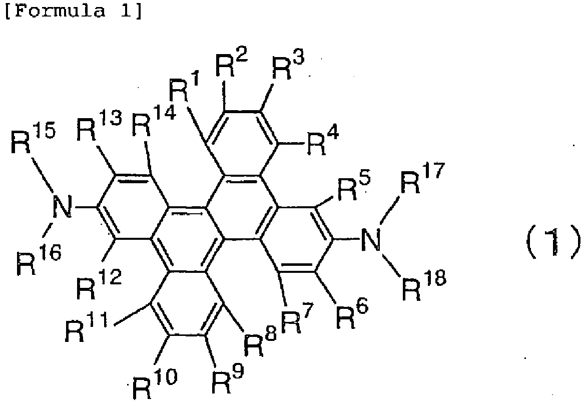 wherein each of R¹ to R¹⁴ is selected from the group consisting of hydrogen, an alkyl group, a cycloalkyl group, an aryl group, a heterocyclic group, a heteroaryl group, an alkenyl group, a cycloalkenyl group, an alkynyl group, an alkoxy group, an alkylthio group, an aryl ether group, an arylthio ether group, a halogen atom, a cyano group, - P(=O)R¹⁹R²⁰ and a silyl group, any two substituents adjacent to each other of R¹ to R¹⁴ may form a ring, R¹⁹ and R²⁰ are respectively an aryl group or a heteroaryl group, R¹⁵ to R¹⁸ may be the same or different and each of them is selected from the group consisting of an alkyl group, a cycloalkyl group, an aryl group, a heterocyclic group and a heteroaryl group and when each of R¹⁵ to R¹⁸ is an aryl group or a heterocyclic group, R¹⁵ and R¹⁶ or R¹⁷ and R¹⁸ may be coupled with each other to form a saturated or an unsaturated ring.

Further, another preferred aspect of the present invention pertains to the light emitting device material used for a light emitting device quoted above,
wherein R¹⁵ to R¹⁸ is an aryl group, and at least one of R¹⁵ to R¹⁸ is represented by the following general formula (4): 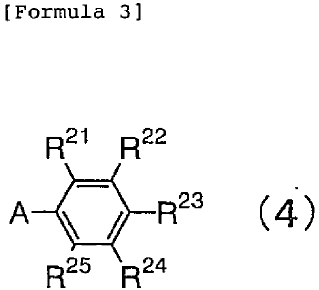 wherein A is used for coupling with a nitrogen atom, each of R²¹ to R²⁵ is selected from the group consisting of hydrogen, an aryl group, an alkyl group, a cycloalkyl group, an alkoxy group, an alkylthio group, an aryl ether group, an arylthio ether group, a silyl group and a fused ring formed between adjacent substituents, provided that at least one of R²¹ to R²⁵ is an aryl group, an alkyl group or a cycloalkyl group, or at least one pair of groups adjacent to each other of R to R²⁵ is a fused ring formed between adjacent substituents.

In accordance with the present invention, a light emitting device having high luminance efficiency, excellent color purity and a long life is obtained.

### Detailed Description of the Preferred Embodiments

A dibenzochrysene compound represented by the general formula (1) will be described in detail.

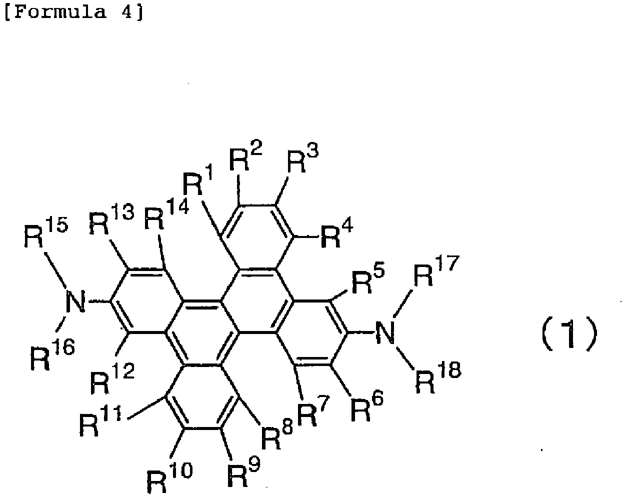

In the formula, R¹ to R¹⁴ may be the same or different and each of them is selected from the group consisting of hydrogen, an alkyl group, a cycloalkyl group, an aryl group, a heterocyclic group, a heteroaryl group, an alkenyl group, a cycloalkenyl group, an alkynyl group, an alkoxy group, an alkylthio group, an aryl ether group, an arylthio ether group, a halogen atom, a cyano group, -P(=O)R¹⁹R²⁰ and a silyl group, any two substituents adjacent to each other of R¹ to R¹⁴ may form a ring, R¹⁹ and R²⁰ are respectively an aryl group or a heteroaryl group, R¹⁵ to R¹⁸ may be the same or different and each of them is selected from the group consisting of an alkyl group, a cycloalkyl group, an aryl group, a heterocyclic group and a heteroaryl group and when each of R¹⁵ to R¹⁸ is an aryl group or a heterocyclic group, R¹⁵ and R¹⁶ or R¹⁷ and R¹⁸ may be coupled with each other to form a saturated or an unsaturated ring.

Among these substituents, the alkyl group represents a saturated aliphatic hydrocarbon group such as a methyl group, an ethyl group, an n-propyl group, an isopropyl group, an n-butyl group, a sec-butyl group or a tert-butyl group, and these alkyl groups may have a substituent or may have no substituent. An additional substituent in the case where the group is substituted is not particularly limited, and examples of the substituent include an alkyl group, an aryl group, and a heteroaryl group. This point is common to the following descriptions. Further, the number of carbon atoms of the alkyl group is not particularly limited, but it is generally preferably 1 or more and 20 or less, and more preferably 1 or more and 8 or less from the viewpoint of ease of availability and cost.

The cycloalkyl group represents a saturated alicyclic hydrocarbon group such as a cyclopropyl group, a cyclohexyl group, a norbornyl group or an adamantly group, and these cycloalkyl groups may have a substituent or may have no substituent. The number of carbon atoms of the cycloalkyl group is not particularly limited, but it is generally preferably 3 or more and 20 or less.

The aryl group represents an aromatic hydrocarbon group such as a phenyl group, a naphthyl group, a biphenyl group, a phenanthryl group, a terphenyl group, a fluorenyl group, an anthracenyl group or a pyrenyl group. The aryl group may have a substituent or may have no substituent. The number of carbon atoms of the aryl group is not particularly limited, but it is generally preferably 6 or more and 40 or less.

The heterocyclic group represents an aliphatic ring which has atoms other than carbon, such as a pyran ring, a piperidine ring and a cyclic amide in the ring, and these heterocyclic groups may have a substituent or may have no substituent. The number of carbon atoms of the heterocyclic group is not particularly limited, but it is generally preferably 2 or more and 20 or less.

The heteroaryl group represents a cyclic aromatic group which has one or plural atoms other than carbon, such as a furanyl group, a thiophenyl group, a pyrrolyl group, a benzofuranyl group, a benzothiophenyl group, a dibenzofuranyl group, a dibenzothiophenyl group, a carbazolyl group, an indolyl group, a pyridyl group and a quinolinyl group in the ring, and these heteroaryl groups may have a substituent or may have no substituent. The number of carbon atoms of the heteroaryl group is not particularly limited, but it is generally preferably 2 or more and 30 or less. The heteroaryl group may be coupled to any position, and in the case of the pyridyl group, any form of a 2-pyridyl group, a 3-pyridyl group and a 4-pyridyl group may be employed.

The alkenyl group represents unsaturated aliphatic hydrocarbon groups containing a double bond such as a vinyl group, an allyl group or a butadienyl group, and these alkenyl groups may have a substituent or may have no substituent. The number of carbon atoms of the alkenyl group is not particularly limited, but it is generally preferably 2 or more and 20 or less.

The cycloalkenyl group represents an unsaturated alicyclic hydrocarbon groups containing a double bond such as a cyclopentenyl group, a cyclopentadienyl group or a cyclohexenyl group, and these cycloalkenyl groups may have a substituent or may have no substituent. The number of carbon atoms of the cycloalkenyl group is not particularly limited, but it is generally preferably 3 or more and 20 or less.

The alkynyl group represents an unsaturated aliphatic hydrocarbon group containing a triple bond such as an ethynyl group, and these alkynyl groups may have a substituent or may have no substituent. The number of carbon atoms of the alkynyl group is not particularly limited, but it is generally preferably within the range of 2 or more and 20 or less.

The alkoxy group represents a functional group formed by binding of aliphatic hydrocarbon groups with one another with an ether bond interposed therebetween such as a methoxy group, an ethoxy group or a propoxy group, and these aliphatic hydrocarbon groups may have a substituent or may have no substituent. The number of carbon atoms of the alkoxy group is not particularly limited, but it is generally preferably 1 or more and 20 or less.

The alkylthio group represents a group formed by substitution of a sulfur atom for the oxygen atom of the ether bond of the alkoxy group. The hydrocarbon group of the alkylthio group may have a substituent or may have no substituent. The number of carbon atoms of the alkylthio group is not particularly limited, but it is generally preferably 1 or more and 20 or less.

The aryl ether group represents a functional group formed by binding of aromatic hydrocarbon groups with one another with an ether bond interposed therebetween such as a phenoxy group, and these aromatic hydrocarbon groups may have a substituent or may have no substituent. The number of carbon atoms of the aryl ether group is not particularly limited, but it is generally preferably 6 or more and 40 or less.

The arylthio ether group represents a group formed by substitution of a sulfur atom for the oxygen atom of the ether bond of the aryl ether group. An aromatic hydrocarbon group in the arylthio ether group may have a substituent or may have no substituent. The number of carbon atoms of the arylthio ether group is not particularly limited, but it is generally preferably 6 or more and 40 or less.

Halogen represents fluorine, chlorine, bromine or iodine.
A cyano group and -P(=O)R¹⁹R²⁰ may have a substituent or may have no substituent. R¹⁹ and R²⁰ are each an aryl group or a heteroaryl group.

The silyl group represents a functional group having a bond to a silicon atom such as a trimethylsilyl group or a triphenylsilyl group, and these silyl groups may have a substituent or may have no substituent. The number of carbon atoms of the silyl group is not particularly limited, but it is generally preferably 3 or more and 20 or less. Further, the number of silicon atoms of the silyl group is generally preferably 1 or more and 6 or less.

An example of the fused ring formed between adjacent substituents, when described with respect to the general formula (2), is a conjugated or nonconjugated fused ring formed between R²¹ and R²². These fused rings may contain a nitrogen, oxygen or sulfur atom in the ring structure, or may be coupled to another ring by condensation.

Among these substituents, hydrogen, an alkyl group, a cycloalkyl group, an aryl group, a heteroaryl group, an alkoxy group, an aryl ether group and a silyl group are preferred as R¹ to R¹⁴. Among others, it is preferred that at least one of R¹ to R¹⁴ is a sterically bulky substituent from the viewpoint of improvement in luminance efficiency and improvement in color purity, and for example, substituents such as an isopropyl group, a tert-butyl group, a cyclohexyl group, an adamantly group, a trimethylsilyl group and a triphenylsilyl group are preferred. Furthermore, in consideration of ease of synthesis, an isopropyl group and a tert-butyl group are more preferred.

The dibenzochrysene compound represented by the general formula (1) of the present invention is a light emitting device material which enables high luminance efficiency and excellent durability by introduction of two amino groups into a specific position of a dibenzochrysene skeleton. That is, the dibenzochrysene compound represented by the general formula (1) exhibits excellent performance such as high luminance efficiency, high color purity and a long life since the substitution position of the amino group is position-2 and position-10 of dibenzochrysene.

Herein, the term light emitting device material refers to a compound involved in the emission in the light emitting device, and corresponds to either of a substance emitting light by itself and a substance assisting such a self-emitting substance to emit light. Specifically, a hole injection material, a hole transporting material, an emissive material, an electron injection material and an electron transporting material correspond to the light emitting device material. Since the dibenzochrysene compound represented by the general formula (1) has excellent heat resistance and a high fluorescence quantum yield and exhibits intense emission in the blue region, it is suitably used particularly as a blue dopant material. By using the dibenzochrysene compound represented by the general formula (1) as the dopant material, it becomes possible to attain a light emitting device having high luminance efficiency and excellent durability.

Further, since the dibenzochrysene compound represented by the general formula (1) has a dibenzochrysene skeleton, which is a large π-electron plane, as a main skeleton, a large hole transporting ability can be expected. Accordingly, the dibenzochrysene compound can be used as a hole transporting material or as a hole injection material. Furthermore, since the ionization potential in a state of an amorphous film of these compounds is about 5.0 to 5.6 eV, these compounds can be more suitably used as the hole injection material.

The emissive material is required to be free from concentration quenching due to the association between molecules. Further, the hole injection material is required to have a high glass transition temperature from the viewpoint of heat resistance and to have an appropriate ionization potential and a high ability to inject and transport a hole. Therefore , R¹⁵ to R¹⁸ in the dibenzochrysene compound represented by the general formula (1) are each preferably an aryl group, and at least one of R¹⁵ to R¹⁸ is preferably a group represented by the general formula (2).

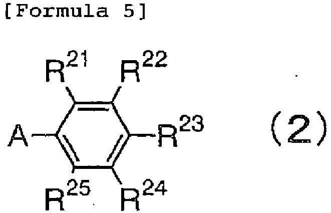

In the formula, A is used for coupling with a nitrogen atom, each of R²¹ to R²⁵ is selected from the group consisting of hydrogen, an aryl group, an alkyl group, a cycloalkyl group, an alkoxy group, an alkylthio group, an aryl ether group, an arylthio ether group, a silyl group and a fused ring formed between adjacent substituents, provided that at least one of R²¹ to R²⁵ is an aryl group, an alkyl group or a cycloalkyl group, or at least one pair of groups adjacent to each other of R²¹ to R²⁵ is a fused ring formed between adjacent substituents.

In addition, in the compound represented by the general formula (1), a compound in which at least one of R¹⁵ to R¹⁸ is represented by the general formula (2) is synonymous with a compound represented by the following general formula (3).

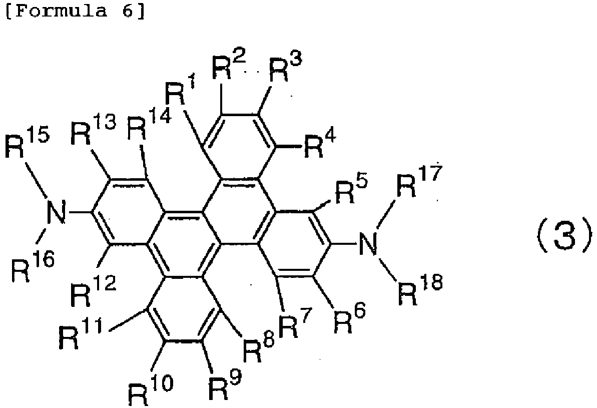

In the formula, R¹ to R¹⁴ may be the same or different and each of them is selected from the group consisting of hydrogen, an alkyl group, a cycloalkyl group, an aryl group, a heterocyclic group, a heteroaryl group, an alkenyl group, a cycloalkenyl group, an alkynyl group, an alkoxy group, an alkylthio group, an aryl ether group, an arylthio ether group, a halogen atom, a cyano group , -P(=O)R¹⁹R²⁰ and a silyl group, any two substituents adjacent to each other of R¹ to R¹⁴ may form a ring, R¹⁹ and R²⁰ are respectively an aryl group or a heteroaryl group, R¹⁵ to R¹⁸ may be the same or different and each of them is selected from the group consisting of an alkyl group, a cycloalkyl group, an aryl group, a heterocyclic group and a heteroaryl group and when each of R¹⁵ to R¹⁸ is an aryl group or a heterocyclic group, R¹⁵ and R¹⁶ or R¹⁷ and R¹⁸ may be coupled with each other to form a saturated or unsaturated ring. Furthermore, at least one of R¹⁵ to R¹⁸ is represented by the following general formula (4): 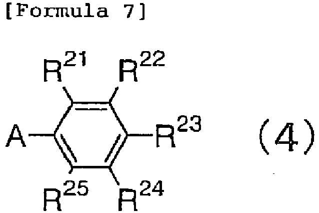 wherein A is used for coupling with a nitrogen atom, each of R²¹ to R²⁵ is selected from the group consisting of hydrogen, an aryl group, an alkyl group, a cycloalkyl group, an alkoxy group, an alkylthio group, an aryl ether group, an arylthio ether group, a silyl group and a fused ring formed between adjacent substituents, provided that at least one of R²¹ to R²⁵ is an aryl group, an alkyl group or a cycloalkyl group, or at least one pair of groups adjacent to each other of R to R²⁵ is a fused ring formed between adjacent substituents.

When at least one of R²¹ to R²⁵ is an aryl group, an alkyl group or a cycloalkyl group, or at least one pair of groups adjacent to each other of R²¹ to R²⁵ is a fused ring formed between adjacent substituents, the glass transition temperature of the dibenzochrysene compound represented by the general formula (1) or (3) is improved and more excellent heat resistance can be achieved. Further, these groups function as substituents of steric hindrance. Therefore, when the compound represented by the general formula (1) or (3) is used as a dopant, if the compound has such a substituent, the association between molecules of the dibenzochrysene compound represented by the general formula (1) or (3) or the association of the dibenzochrysene compound with a host compound is inhibited, concentration quenching and degradation of color purity can be suppressed, and a device having higher luminance efficiency can be obtained.

An aryl group, an alkyl group and a cycloalkyl group are electron-donating groups and when at least one pair of groups adjacent to each other of R²¹ to R²⁵ is a fused ring formed between adjacent substituents, the groups are also electron-donating groups. Therefore, when the compound represented by the general formula (1) or (3) is used as a hole injection material, if the compound has such a substituent, the ionization potential of the dibenzochrysene compound represented by the general formula (1) or (3) is decreased and therefore a hole is smoothly injected from an anode. As a result of this, the driving voltage of the light emitting device is reduced and consequently the effect of lengthening the life of the light emitting device is achieved.

When the compound represented by the general formula (1) or (3) is used as a dopant, in the general formulas (2) and (4), preferred specific examples of the alkyl group or the cycloalkyl group of R²¹ to R²⁵ include alkyl groups such as a methyl group, an ethyl group, an n-propyl group, an isopropyl group, an n-butyl group, a sec-butyl group and a tert-butyl group, and cycloalkyl groups such as a cyclopropyl group, a cyclohexyl group, a norbornyl group and an adamantly group. Among these substituents, a sterically bulky substituent is preferred from the viewpoint of improvement in luminance efficiency and improvement in color purity, and an isopropyl group, a tert-butyl group, a cyclohexyl group, and an adamantly group are preferred. Furthermore, from the viewpoint of ease of availability of the raw material and relative ease of synthesis, an isopropyl group and a tert-butyl group are more preferred.

Specific examples of the aryl groups of R²¹ to R²⁵ are similar to those in the description of the general formula (1). However, since the wavelength of emission becomes long and color purity may deteriorate if an aryl group having the number of the fused rings of 3 or more is introduced, the aryl group of R²¹ to R²⁵ is preferably an aryl group having the number of nucleus carbon atoms of 6 to 18 or an aromatic hydrocarbon group having the number of fused rings of 2 or less. Among these, a phenyl group, a biphenyl group, a fluorenyl group, a terphenyl group, and a naphthyl group are more preferred.

Further, examples of the case where at least one pair of groups adjacent to each other of R²¹ to R²⁵ is a fused ring formed between adjacent substituents include a case where a group represented by the general formula (2) is consequently a naphthyl group, a fluorenyl group, an anthracenyl group or a pyrenyl group. However, since the wavelength of emission becomes long and color purity may deteriorate when the number of the fused rings is 3 or more, the group represented by the general formula (2) is preferably a naphthyl group or a fluorenyl group.

Among these, it is more preferred that at least one of R²¹ to R²⁵ is an alkyl group or a cycloalkyl group in order to particularly preferably improve both of the luminance efficiency and the color purity.

With respect to the substituents described above, a plurality of same substituents may be introduced, or a plurality of species of substituents may be mixed and introduced. Further, in the general formulas (2) and (4), specific examples of the case where R²¹ to R²⁵ are other groups are similar to those in the description of the general formula (1).

On the other hand, when the compound represented by the general formula (1) or (3) is used as a hole injection material, in the general formulas (2) and (4), preferred specific examples of the alkyl group or the cycloalkyl group of R²¹ to R²⁵ include alkyl groups such as a methyl group, an ethyl group, an n-propyl group, an isopropyl group, an n-butyl group, a sec-butyl group and a tert-butyl group, and cycloalkyl groups such as a cyclopropyl group, a cyclohexyl group, a norbornyl group and an adamantly group. Among these substituents, from the viewpoint of the improvement in the glass transition temperature and the reduction in barrier to the hole injection from the anode, a methyl group, an ethyl group, an n-propyl group, an isopropyl group, an n-butyl group, a tert-butyl group, a cyclohexyl group and an adamantly group are preferred. Furthermore, from the viewpoint of ease of availability of the raw material and ease of synthesis, a methyl group, an ethyl group, an isopropyl group and a tert-butyl group are more preferred.

Specific examples of the aryl groups of R²¹ to R²⁵ are similar to those in the description of the general formula (1). However, since the wavelength of absorption becomes long to absorb emission from the emissive layer and luminance efficiency may deteriorate if an aryl group having the number of the fused rings of 3 or more is introduced, the aryl group of R²¹ to R²⁵ is preferably an aryl group having the number of nucleus carbon atoms of 6 to 18 or an aromatic hydrocarbon group having the number of fused rings of 2 or less. Among these, a phenyl group, a biphenyl group, a fluorenyl group, a terphenyl group, and a naphthyl group are more preferred.

Further, examples of the case where at least one pair of groups adjacent to each other of R²¹ to R²⁵ is a fused ring formed between adjacent substituents include a case where a group represented by the general formula (2) is consequently a naphthyl group, a fluorenyl group, an anthracenyl group or a pyrenyl group. However, since the wavelength of absorption becomes long to absorb emission from the emissive layer and luminance efficiency may deteriorate when the number of the fused rings is 3 or more, the group represented by the general formula (2) is preferably a naphthyl group or a fluorenyl group.

With respect to the substituents described above, a plurality of same substituents may be introduced, or a plurality of species of substituents maybemixed and introduced. Further, in the general formulas (2) and (4), specific examples of the case where R²¹ to R²⁵ are other groups are similar to those in the description of the general formula (1).

The dibenzochrysene compound represented by the general formula (1) or (3), described above, is not particularly limited, and specific examples thereof include the following compounds.

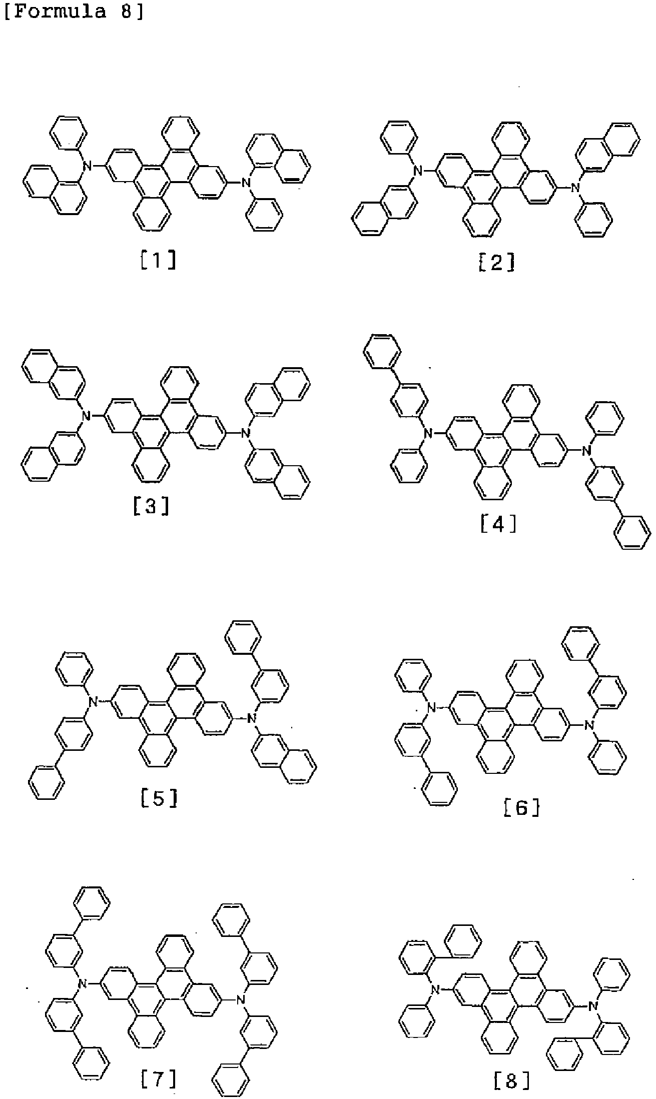

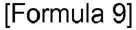

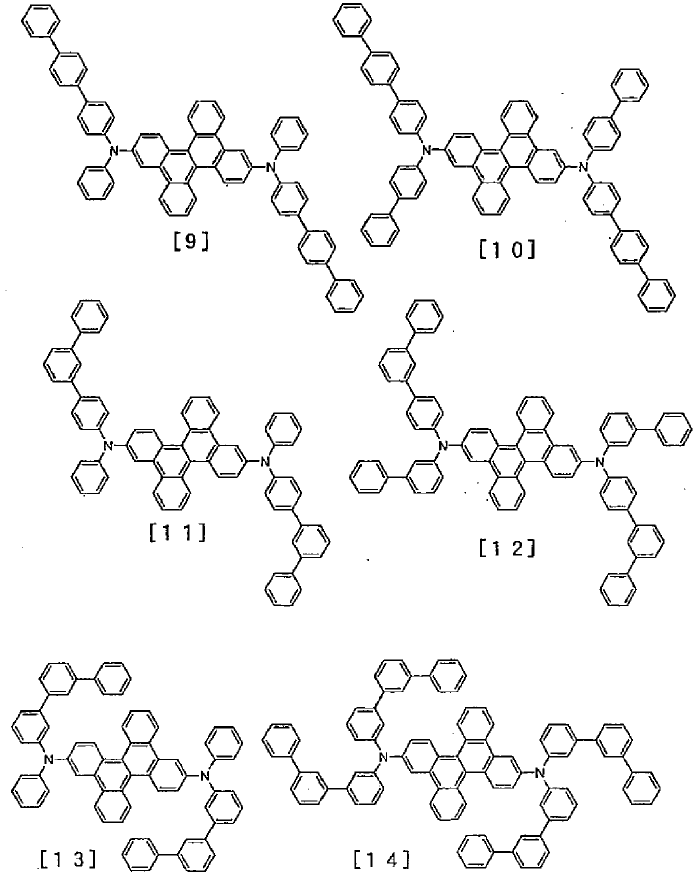

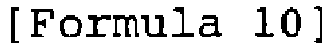

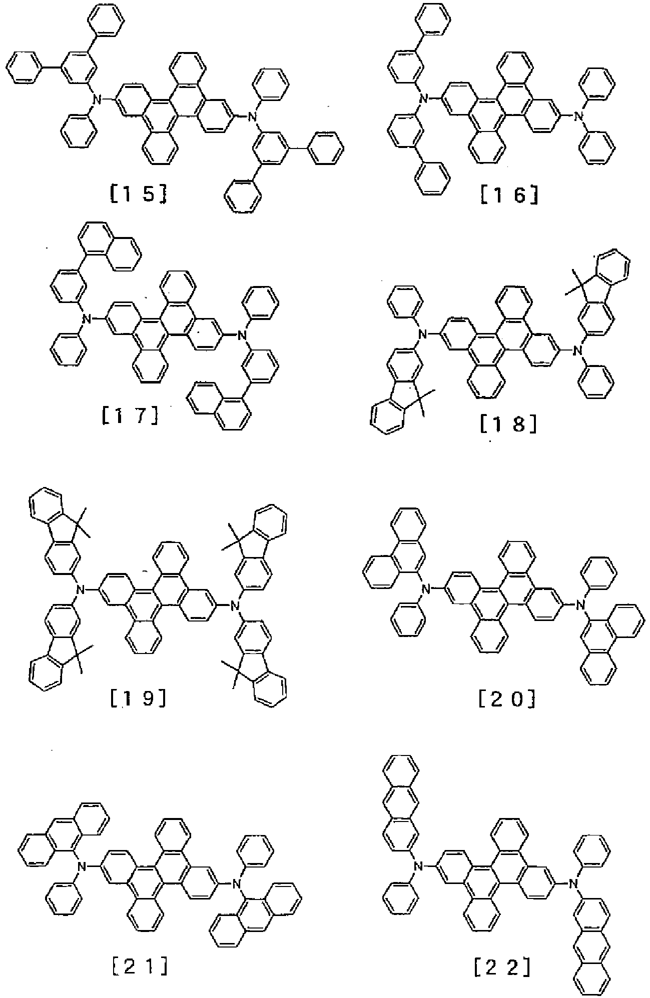

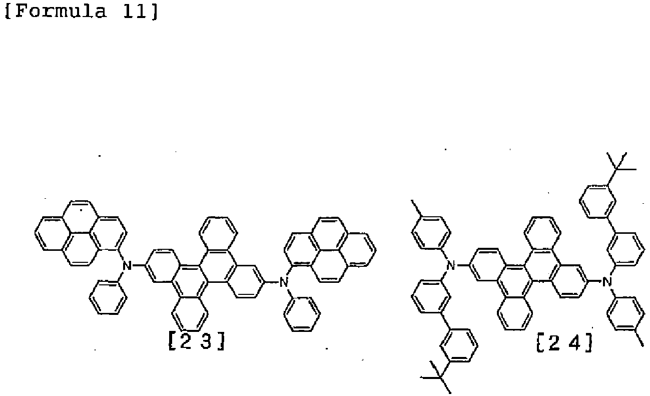

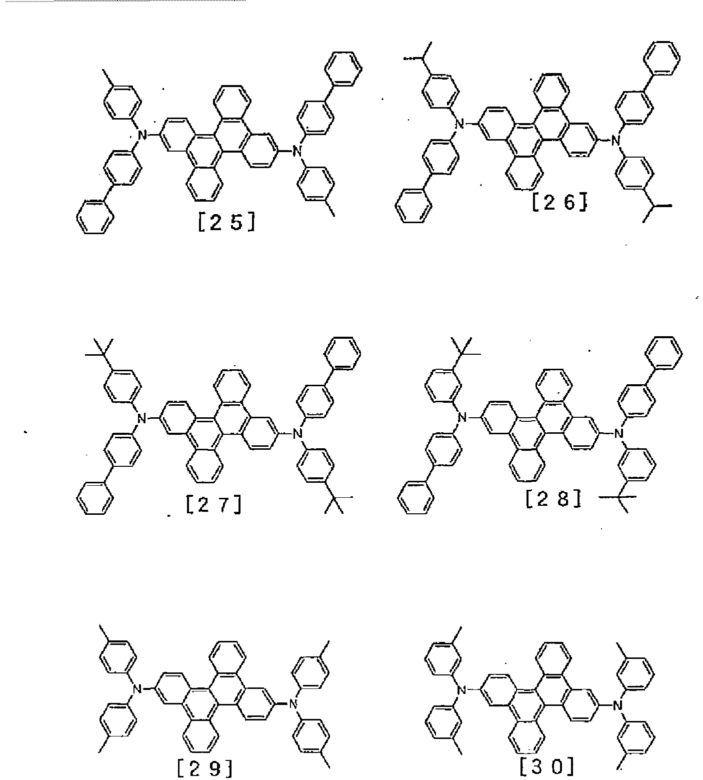

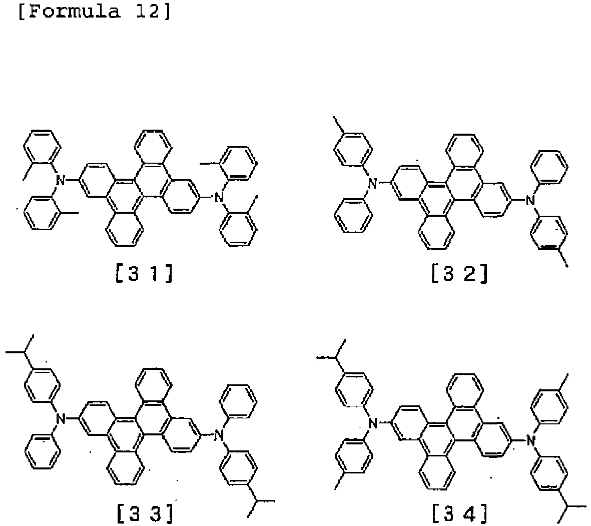

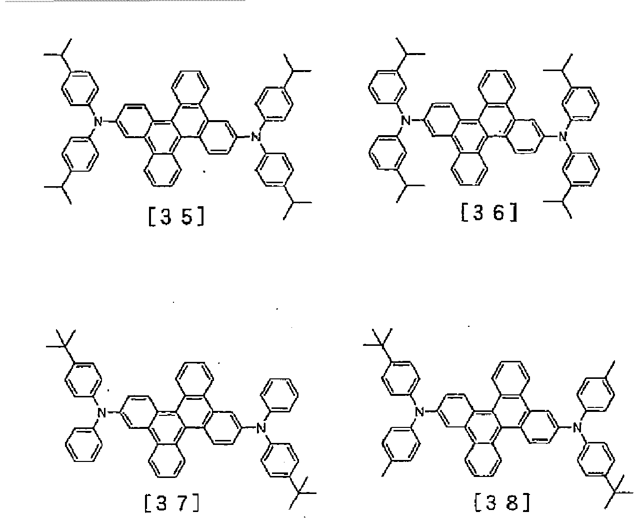

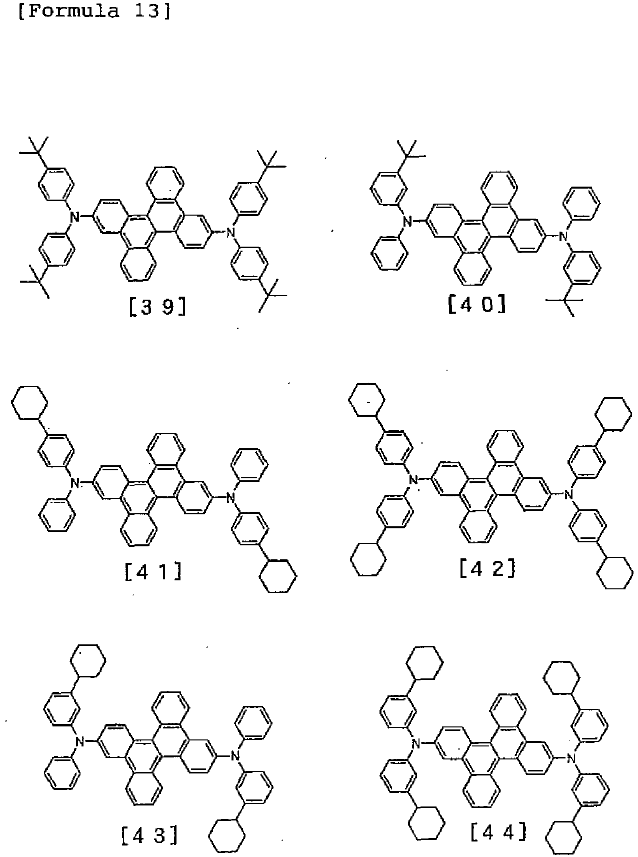

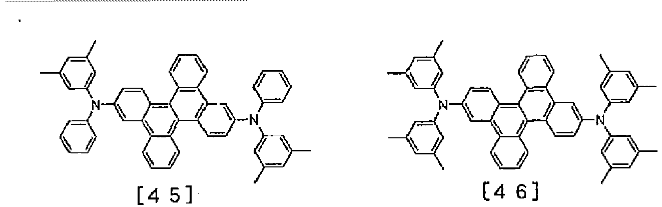

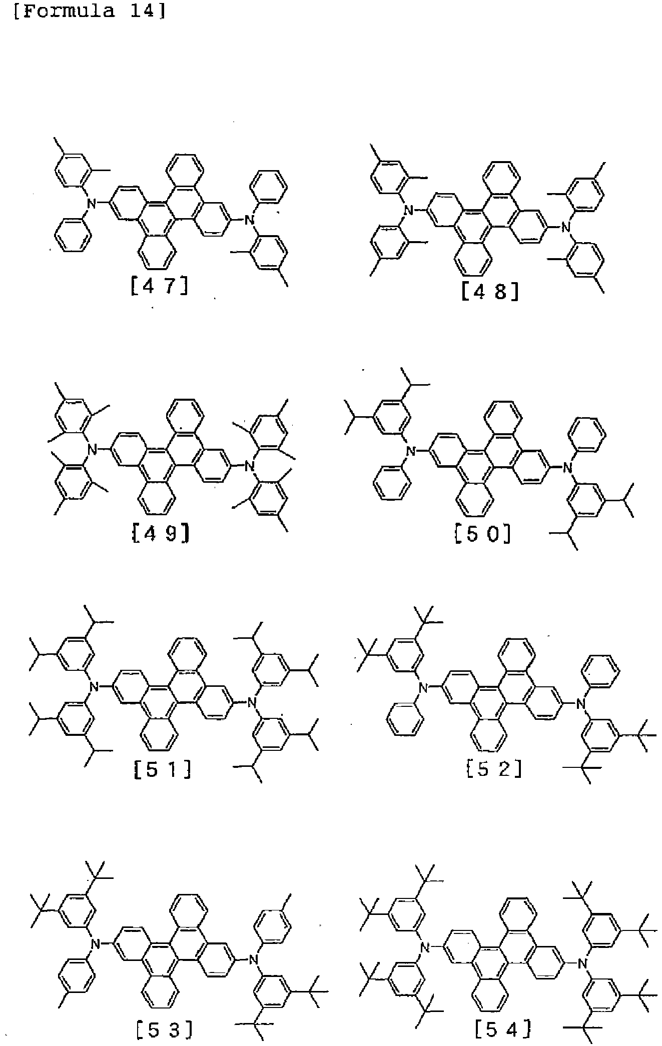

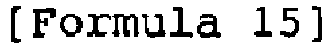

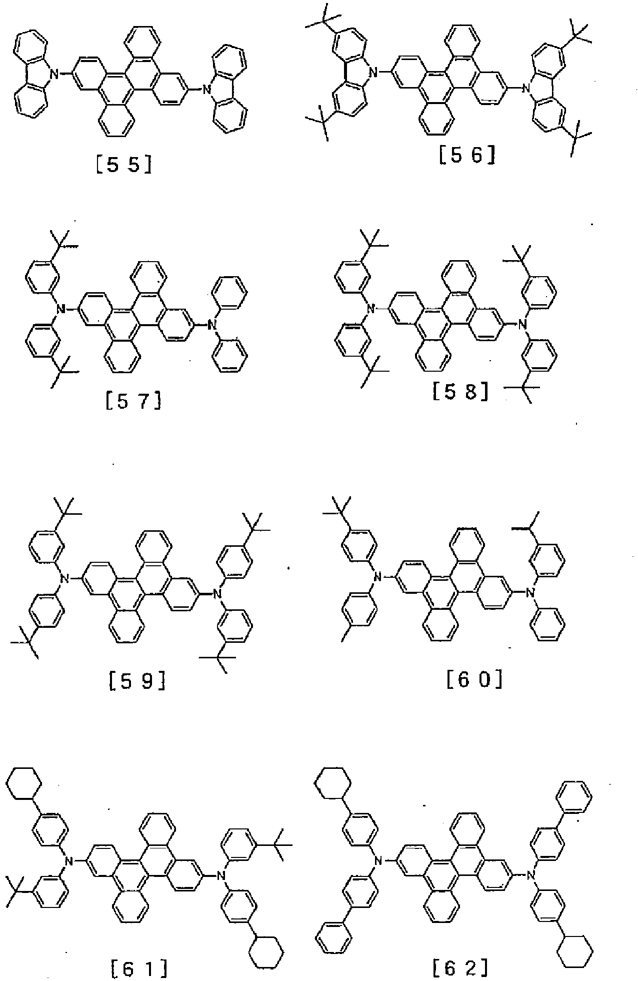

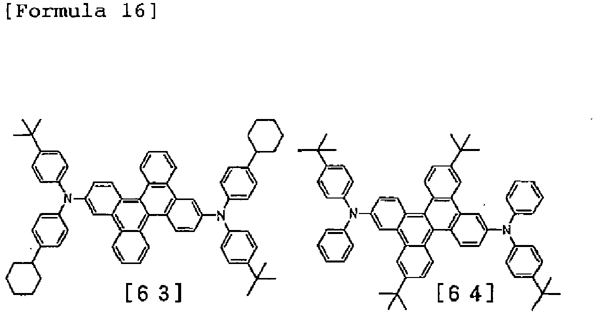

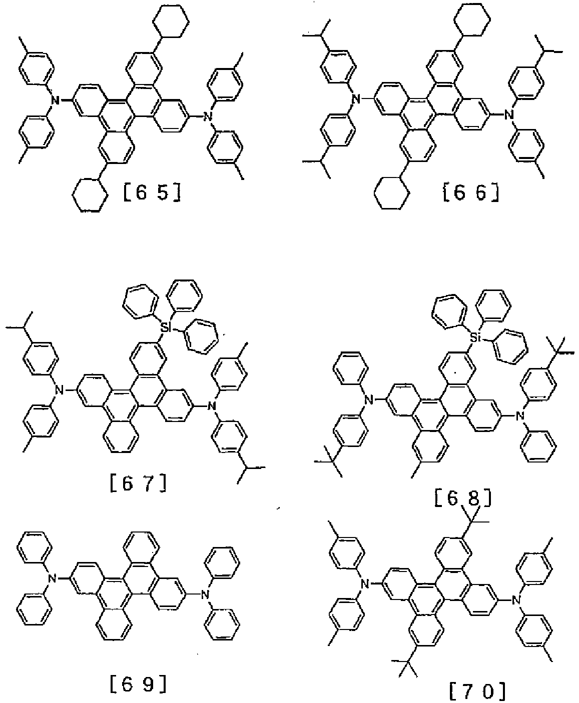

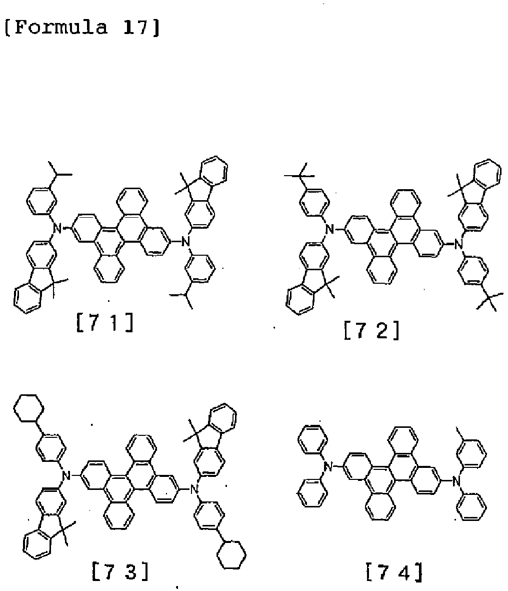

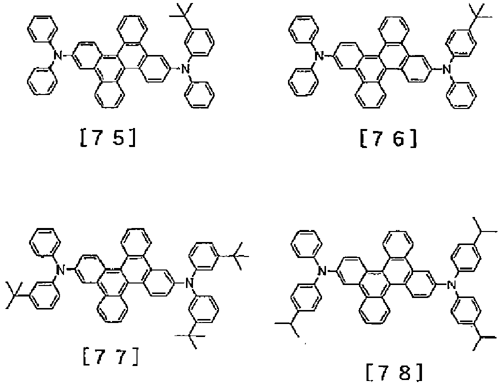

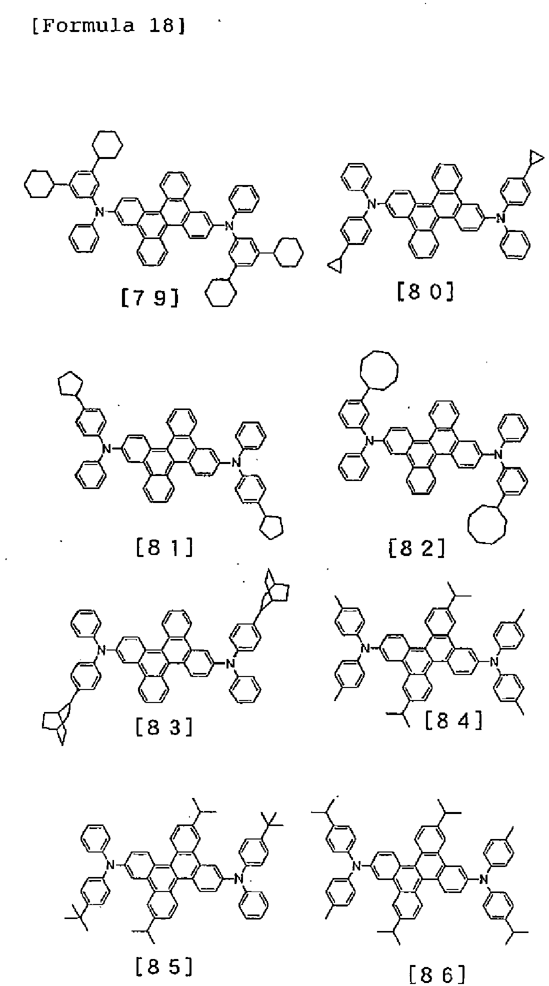

Publicly known methods can be used for synthesizing the compound represented by the general formula (1) or (3). For example, a dimethoxy derivative of dibenzochrysene, which is a main skeleton, can be synthesized by a method described in Journal of American Chemical Society (US), 2001, vol. 123, pp. 12087-12088. Furthermore, the methoxy group may be changed to a hydroxyl group and subsequently to triflate group, and the triflate derivative can be reacted with diarylamine by a coupling reaction using a palladium catalyst to obtain an objective compound, but the synthesis method of the compound is not limited to this method.

Next, embodiments of the light emitting device of the present invention will be described. The light emitting device of the present invention has an anode, a cathode, and an organic layer present there between, the organic layer contains at least an emissive layer, or contains a hole injection layer and an emissive layer, and the emissive layer emits light by electrical energy. In the light emitting device of the present invention, the dibenzochrysene compound represented by the general formula (1) or (3) is contained in the organic layer.

Examples of the constitution of the organic layer include lamination constitutions such as a hole transporting layer/an emissive layer, a hole transporting layer/an emissive layer/an electron transporting layer, a hole injection layer/a hole transporting layer/an emissive layer, and a hole injection layer/a hole transporting layer/an emissive layer/an electron transporting layer in addition to a constitution consisting only of an emissive layer. Further, the above-described respective layers may be composed of a single layer or multiple layers. When the electron transporting layer is composed of multiple layers, the layer on the side in contact with an electrode may be referred to as an electron injection layer, and the electron injection layer is included in the electron transporting layer in the following description.

The material of the anode is not particularly limited as long as it is a material with which a hole can be efficiently injected into the organic layer, but a material having a relatively large work function is preferably used. Examples of the anode material include conductive metal oxides such as tin oxide, indium oxide, indium zinc oxide and indium tin oxide (ITO), metals such as gold, silver and chromium, inorganic conductive substances such as copper iodide and copper sulfide, and conductive polymers such as polythiophene, polypyrrole and polyaniline. These electrode materials may be used alone or by laminating or mixing a plurality of materials.

The resistance of the anode is only required to supply a current sufficient for the emission of a light emitting device, but it is desirably low from the viewpoint of the power consumption of the light emitting device. For example, when the resistance is 300 Ω/square or lower, the anode functions as an electrode, but it is particularly desirable to use a product with a low resistance of 100 Ω/square or lower. The thickness of the anode can be arbitrarily selected in accordance with the resistance value, but an anode of 100 to 300 nm in thickness is usually used.

Further, in order to maintain the mechanical strength of the light emitting device, the anode is preferably formed on a substrate. As the substrate, a substrate of glass such as soda glass or alkali-free glass is suitably used. As the thickness of the glass substrate, a thickness of 0.5 mm or more is enough since the glass substrate is only required to have an adequate thickness for maintaining the mechanical strength. The material of glass is more preferably alkali-free glass since less ion elution from glass is more preferable, but commercially available soda lime glass coated with a barrier coat such as SiO₂ can also be used. Further, the material of the substrate does not have to be glass as long as the anode operates stably, and for example, the anode may be formed on a plastic substrate. The method of forming the anode is not particularly limited and, for example, an electron beam method, a sputtering method and a chemical reaction method can be employed.

The material to be used for a cathode is not particularly limited as long as it is a material with which an electron can be efficiently injected into the organic layer, and examples of the material include platinum, gold, silver, copper, iron, tin, zinc, aluminum, indium, chromium, lithium, sodium, potassium, cesium, calcium and magnesium, and alloys thereof. In order to enhance the electron injection efficiency to improve device characteristics, lithium, sodium, potassium, cesium, calcium and magnesium, or alloys containing these metals of a low work function are effective. However, since these metals of a low work function are often unstable in the air, preferred examples of method for obtaining the cathode include a method for obtaining a highly stable electrode by doping the organic layer with a trace amount (the film thickness by vacuum evaporation is 1 nm or less in terms of a readout by a film thickness meter) of lithium or magnesium. Further, it is also possible to use inorganic salts such as lithium fluoride. Furthermore, it can be mentioned as a preferred example to laminate metals such as platinum, gold, silver, copper, iron, tin, aluminum or indium or alloys using these metals, inorganic substances such as silica, titania or silicon nitride, and organic polymer compounds such as polyvinyl alcohol, polyvinyl chloride or hydrocarbon-based polymer compounds in order to protect the electrode. The method of forming the cathode is not particularly limited and for example, a resistance heating method, an electron beam method, a sputtering method, an ion plating method and a coating method can be used.

A hole injection layer is formed by a method of laminating or mixing one species or two or more species of hole injection materials, or a method using a mixture of the hole injection material and a polymer binder. Further, the hole injection layer may be formed by adding an inorganic salt such as iron chloride (III) to the hole injection material. Further, a metal oxide such as molybdenum oxide or vanadium oxide may be added to the hole injection material to form a hole injection layer. Furthermore, the hole injection layer can also be formed by adding or laminating a compound having a strong accepting property such as a cyano group-substituted aromatic aza compound. Further, a polymer having a function of injecting and transporting a hole may be dissolved in a solvent and a thin film may be formed from the resulting solution by a spin coating method or an ink-jet method. In this case, a Lewis acid such as iron chloride (III) or antimony chloride (V) may be added to the solution. The hole injection material is not particularly limited as long as it is a compound which can form a thin film and can transport a hole, and into which the hole can be smoothly injected from the anode. As the hole injection material, for example, diphenylbenzidine derivatives such as 4,4'-bis(N-(3-methylphenyl)-N-phenylamino)biphenyl and 4,4'-bis(N-(1-naphthyl)-N-phenylamino)biphenyl, triphenylamine derivatives such as 4,4',4" -tris(3-methylphenyl(phenyl)amino)triphenylamine, biscarbazole derivatives such as bis(N-arylcarbazole) and bis(N-alkylcarbazole), heterocyclic compounds such as pyrazoline derivatives, stilbene compounds, hydrazone compounds, benzofuran derivatives, thiophene derivatives, oxadiazole derivatives, phthalocyanine derivatives and porphyrin derivatives, and polymers such as polycarbonate and styrene derivatives having the above-described monomers on the side chains, polythiophene, polyaniline, polyfluorene, polyvinylcarbazole and polysilane are preferred.

When the dibenzochrysene compound represented by the general formula (1) or (3) of the present invention is used as a hole injection material, only one species of the dibenzochrysene compound may be used, or plural species of the dibenzochrysene compounds may be mixed and used. Further, the same technique as in the description of the hole injection layer described above, that is, the technique of mixing the metal oxide, the technique of adding or laminating a compound having a strong accepting property such as a cyano group-substituted aromatic aza compound, and the technique of adding a Lewis acid can be employed.

A hole transporting layer is formed by a method of laminating or mixing one species or two or more species of hole transporting materials, or a method using a mixture of the hole transporting material and a polymer binder. Further, the hole transporting material is not particularly limited as long as it is a compound which can form a thin film and can transport a hole, and into which the hole can be injected from the anode or the hole injection layer. As the hole transporting material, for example, diphenylbenzidine derivatives such as 4,4'-bis(N-(3-methylphenyl)-N-phenylamino)biphenyl and 4,4'-bis(N-(1-naphthyl)-N-phenylamino)biphenyl, triphenylamine derivatives such as 4,4',4" -tris(3-methylphenyl(phenyl)amino)triphenylamine, biscarbazole derivatives such as bis(N-arylcarbazole) and bis(N-alkylcarbazole), heterocyclic compounds such as pyrazoline derivatives, stilbene compounds, hydrazone compounds, benzofuran derivatives, thiophene derivatives and porphyrin derivatives, and polymers such as polycarbonate and styrene derivatives, having the above-described monomers on the side chains, polythiophene, polyaniline, polyfluorene, polyvinylcarbazole and polysilane are preferred.

The emissive layer may be composed of a single layer or multiple layers, and both of the single layer and the multiple layers may be formed from a mixture of a host material and a dopant material or a host material alone. The host material and the dopant material may be respectively consist of one species or a plurality of species. The dopant material may be contained in the whole host material or may be partially contained in the host material. The dopant material and the host material may be laminated or the dopant material may be dispersed in the host material. Since the concentration quenching takes place when the amount of the dopant material is too large, this amount is preferably 20% by weight or less with respect to the total of the host material and the dopant material. As the doping method, the dopant material may be formed by co-evaporation of the dopant material and the host material, or the host material and the dopant material may be previously mixed before evaporation.

The dibenzochrysene compound of the present invention may be used as a host material, but it is suitably used as a dopant material because of a high fluorescence quantum yield. The ionization potential of the dibenzochrysene compound represented by the general formula (1) or (3) of the present invention is not particularly limited, but the ionization potential is preferably 5 eV or more and 7 eV or less, more preferably 5.0 eV or more and 5.9 eV or less, and furthermore preferably 5.0 eV or more and 5.6 eV or less.

Although it is reported that the absolute value of the ionization potential varies depending on the measuring method, the value of the ionization potential in the present invention was obtained by measuring a thin film, deposited on an ITO glass substrate in a thickness of 30 to 100 nm, with an atmospheric type ultraviolet photoelectron analyzing apparatus (AC-1, manufactured by RIKENKEIKI CO., LTD.).

As the dopant material, one species of the dibenzochrysene compound represented by the general formula (1) or (3) may be used, or plural species of the dibenzochrysene compounds may be mixed and used. Further, one or more species of other dopant materials may be mixed with the dibenzochrysene compound represented by the general formula (1) or (3). Examples of the dopant materials which can be mixed with the dibenzochrysene compound include compounds having an aryl ring such as naphthalene, anthracene, phenanthrene, pyrene, triphenylene, perylene, fluorene and indene, and derivatives thereof (for example, 2-(benzothiazole-2-yl)-9,10-diphenylanthracene and 5,6,11,12-tetraphenylnaphthacene); compounds having a heteroaryl ring such as furan, pyrrole, thiophene, silole, 9-silafluorene, 9,9'-spirobisilafluorene, benzothiophene, benzofuran, indole, dibenzothiophene, dibenzofuran, imidazopyridine, phenanthroline, pyrazine, naphthylidine, quinoxaline, pyrrolopyridine and thioxanthen, and derivatives thereof; distyrylbenzene derivatives; aminostyryl derivatives such as 4,4'-bis(2-(4-diphenylaminophenyl)ethenyl)biphenyl and 4,4'-bis(N-(stilbene-4-yl)-N-phenylamino)stilbene; aromatic acetylene derivatives; tetraphenylbutadiene derivatives; stilbene derivatives; aldazine derivatives; pyrromethene derivatives; diketopyrrolo[3,4-c]pyrrole derivatives; coumarin derivatives such as 2.,3,5,6-1H,4H-tetrahydro-9-(2'-benzothiazolyl)quinolizino[9 ,9a,1-gh]coumarin; azole derivatives such as imidazole, thiazole, thiadiazole, carbazol, oxazole, oxadiazole and triazole, and metal complexes thereof; and aromatic amine derivatives typified by N,N'-diphenyl-N,N'-di(3-methylphenyl)-4,4'-diphenyl-1,1'-di amine.

When the dibenzochrysene compound represented by the general formula (1) or (3) is used as the dopant material, the concentration of doping is preferably 20% by weight or less in order to suppress the concentration quenching, as described above. On the other hand, when the concentration of doping is as low as 1% by weight or less, there is a possibility of adverse influence on the durability life of the device and therefore, it is more preferred to use the dibenzochrysene compound in a concentration in the range of 1 to 20% by weight.

The host material contained in the emissive material is not particularly limited, but compounds having a condensed aryl ring such as anthracene and pyrene and derivatives thereof, aromatic amine derivatives such as N,N'-dinaphthyl-N,N'-diphenyl-4,4'-diphenyl-1,1'-diamine, metal-chelated oxinoid compounds including tris(8-guinolinato)aluminum (III), bisstyryl derivatives such as distyrylarylene derivatives, tetraphenylbutadiene derivatives, indene derivatives, coumarin derivatives, oxadiazole derivatives, pyrrolopyridine derivatives, perinone derivatives, cyclopentadiene derivatives, oxadiazole derivatives, carbazole derivatives, pyrrolopyrrole derivatives, and polymers such as polyphenylenevinylene derivatives, poly(p-phenylene) derivatives, polyfluorene derivatives, polyvinylcarbazole derivatives, and polythiophene. derivatives are suitably used. Among others, when an aromatic hydrocarbon derivative having an electron-donating property or a neutral substituent is used as a host, it is preferred because the effect of high luminance efficiency of the dibenzochrysene compound of the present invention is exhibited more remarkably. Specifically, when a compound selected from anthracene compounds, pyrene compounds and distyrylarylene compounds is used as a host material, it is preferred because the luminance efficiency is improved by a combination of this compound and the dibenzochrysene compound of the present invention. When an anthracene compound or a pyrene compound having higher heat resistance and carrier-transporting capacity is combined with the dibenzochrysene compound of the present invention, it is more preferred since a light emitting device having high efficiency and a long life is obtained.

The electron transporting layer is a layer into which an electron is injected from the cathode and which further transports the electron. The material to be used for the electron transporting layer is not particularly limited as long as it is a compound which has high electron injection efficiency and transports the injected electron efficiently. Specific examples of the material to be used for the electron transporting layer include compounds having a condensed aryl ring such as naphthalene and anthracene, and derivatives thereof; styryl-based aromatic ring derivatives typified by 4,4'-bis(diphenylethenyl)biphenyl, perylene derivatives, perynone derivatives, coumarin derivatives, naphthalimide derivatives, quinone derivatives such as anthraquinone and diphenoquinone, phosphorus oxide derivatives, carbazole derivatives and indole derivatives, quinolinol complexes such as tris(8-quinolinolato)aluminum(III), hydroxyazole complexes such as hydroxyphenyloxazole complex, azomethine complexes, tropolone metal complexes and flavonol metal complexes . As the electron transporting material, it is preferred to use a compound having a heteroaryl ring structure which is composed of elements selected from carbon, hydrogen, nitrogen, oxygen, silicon, and phosphorus and includes electron-accepting nitrogen since the driving voltage can be reduced.

The electron-accepting nitrogen refers to a nitrogen atom which forms a multiple bond between itself and an adjacent atom. Since the nitrogen atom has high electronegativity, the multiple bond has a property like an electron-accepting property and an excellent electron transporting capacity, and therefore by using the multiple bond for the electron transporting layer, the driving voltage of the light emitting device can be reduced. Thus, the heteroaryl ring including the electron-accepting nitrogen has high electron affinity. Examples of the heteroaryl ring including the electron-accepting nitrogen include a pyridine ring, a pyrazine ring, a pyrimidine ring, a quinoline ring, a quinoxaline ring, a naphthyridine ring, a pyrimidopyrimidine ring, a benzoquinoline ring, a phenanthroline ring, an imidazole ring, an oxazole ring, an oxadiazole ring, a triazole ring, a thiazole ring, a thiadiazole ring, a benzoxazole ring, a benzothiazole ring, a benzimidazole ring, and a phenanthroimidazole ring.

Examples of preferred compounds having the heteroaryl ring structure include benzimidazole derivatives, benzoxazole derivatives, benzothiazole derivatives, oxadiazole derivatives, thiadiazole derivatives, triazole derivatives, pyrazine derivatives, phenanthroline derivatives, quinoxaline derivatives, quinoline derivatives, benzoquinoline derivatives, oligopyridine derivatives such as bipyridine and terpyridine, quinoxaline derivatives, and naphthyridine derivatives. Among these compounds, imidazole derivatives such as tris(N-phenylbenzimidazole-2-yl)benzene, oxadiazole derivatives such as 1,3-bis[(4-tert-butylphenyl)1,3,4-oxadiazoryl]phenylene, triazole derivatives such as N-naphthyl-2,5-diphenyl-1,3,4-triazole, phenanthroline derivatives such as bathocuproin and
1,3-bis(1,10-phenanthroline-9-yl)benzene, benzoquinoline derivatives such as 2,2'-bis(benzo[h]quinoline-2-yl)-9,9'-spirobifluorene, bipyridine derivatives such as 2,5-bis(6'-(2',2"-bipyridyl))-1,1-dimethyl-3,4-diphenylsil ole, terpyridine derivatives such as
1,3-bis(4'-(2,2':6'2"-terpyridinyl))benzene, and naphthyridine derivatives such as bis(1-naphthyl)-4-(1,8-naphthyridine-2-yl)phenylphosphineox ide are preferably used from the viewpoint of a electron transporting capacity. Particularly preferred examples include phenanthroline dimers such as
1,3-bis(1,10-phenanthroline-9-yl)benzene, 2,7-bis(1,10-phenanthroline-9-yl)naphthalene and 1,3-bis(2-phenyl-1,10-phenanthroline-9-yl)benzene, and bipyridine dimers such as 2,5-bis(6'-(2',2"-bipyridyl))-1,1-dimethyl-3,4-diphenylsil ole since the effect of improving luminance efficiency is significantly high when these dimers are combined with an emissive layer containing the dibenzochrysene compound represented by the general formula (1).

The above-described electron transporting materials may be used singly or as a mixture of two or more species thereof, or one or more other electron transporting materials may be mixed with the above-described electron transporting material. Further, the electron transporting material can be mixed with a metal such as an alkali metal or an alkaline earth metal. The ionization potential of the electron transporting layer is not particularly limited, but it is preferably 5.8 eV or more and 8.0 eV or less, and more preferably 6.0 eV or more and 7.5 eV or less.

The method of forming the respective layers constituting the light emitting device is not particularly limited and examples thereof include a resistance heating evaporation method, an electron beam evaporation method, asputteringmethod, a molecular lamination method, a coating method, an ink-jet method, a printing method, and a laser-induced thermal transfer method. Usually, the resistance heating evaporation method or the electron beam evaporation method is preferred from the viewpoint of device characteristics.

The thickness of each of the layers cannot be limited since it depends on the resistance of the emissive substance, but it is selected from the range of 1 to 1000 nm. Film thicknesses of the emissive layer, the electron transporting layer and the hole transporting layer are respectively preferably 1 nm or more and 200 nm or less, and more preferably 5 nm or more and 100 nm or less.

The light emitting device of the present invention has a function of converting electrical energy to light. Herein, as the electrical energy, a direct current is mainly used but a pulse current or an alternating current can also be used. The values of the current and the voltage are not particularly limited, but they are preferably selected so as to attain the maximum luminance with as low energy as possible in consideration of the power consumption and the life of the device.

The light emitting device of the present invention is suitably used, for example, as displays displayed in a matrix system and/or segment system. In the matrix system, pixels for display are arrayed in a two-dimensional form such as a grid form or a mosaic form and a set of the pixels display characters and images. The shape and size of the pixel is determined according to the use. For example, for displaying images and characters on a personal computer, a monitor or a TV set, square pixels having a side of 300 µm or smaller are generally used, and in the case of a large display such as a display panel, pixels having a side of the order of millimeter are used. In the case of monochrome display, pixels having the same color may be arrayed, but in the case of color display, red, green and blue pixels are arrayed for display. In the case of color display, a typical array system is a delta type or a stripe type. The method for driving this matrix may be either of passive matrix driving and active matrix driving. In the passive matrix driving, the structure of the light emitting device is simple, but there may be cases where the active matrix driving is superior to the passive matrix driving in consideration of the operational characteristics. The driving method is switched depending on the application.

The segment system is a system in which a pattern is formed so as to display predetermined information and an area defined by the layout of the pattern is made to emit light. Examples of this system include time indications or temperature indications in a digital clock or a digital thermometer, display of operational state of audio equipment or electromagnetic cookware, and an automobile panel display. Further, the matrix display and the segment display may co-exist in the same panel.

The light emitting device of the present invention is also preferably used as backlights of various equipment. The backlight is predominantly used for the purpose of improving the visibility of a display which does not emit light spontaneously and used for a liquid crystal display, a clock, audio equipment, an automobile panel, a display board, and a sign. The light emitting device of the present invention is preferably used for a backlight for liquid crystal displays, particularly a backlight for personal computers for which thinning is investigated. With the light emitting device of the present invention, it is possible to provide a backlight of low-profile and lighter than the conventional backlights.

### Examples

Hereinafter, the present invention will be described by way of examples, but the present invention is not limited to these examples. The numbers of compounds in the following examples refer to the numbers of the compounds shown in the above-described chemical formulas. Further, ¹H-NMR was measured by use of superconducting FT-NMR EX-270 (manufactured by JEOL Ltd.) using a deuterated chloroform solution. Synthesis Example 1 (Synthesis of Compound [29])

### (1-1) Synthesis of Intermediate A

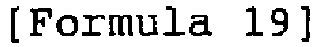

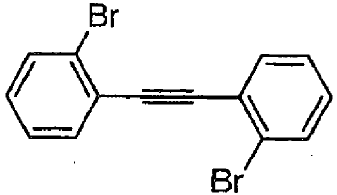

### Intermediate A

Into a 1 liter four-necked flask whose inside was replaced with a nitrogen gas, 35 g of 2-bromo-iodebenzene, 5.8 g of trimethylsilylacetylene, 3.5 g of bis(triphenylphosphine)palladium(II)dichloride, 3.4 g of copper iodide, 100 g of 1,8-diazabicyclo[5,4,0]-7-undecene, 6 ml of distilled water, and 400 ml of toluene were charged, and the resulting mixture was reacted at 60°C for 18 hours. After the completion of the reaction, separation/washing of the reaction liquidwas carried out five times with 400 ml of distilled water. The resulting organic layer was dried over magnesium sulfate and then the solvent was distilled off using a rotary evaporator. The resulting condensed product was purified by silica gel chromatography and recrystallized with hexane and then the crystal was vacuum-dried to obtain 13.3 g of the desired product (white crystal, yield 67.2%).

### (1-2) Synthesis of Intermediate B

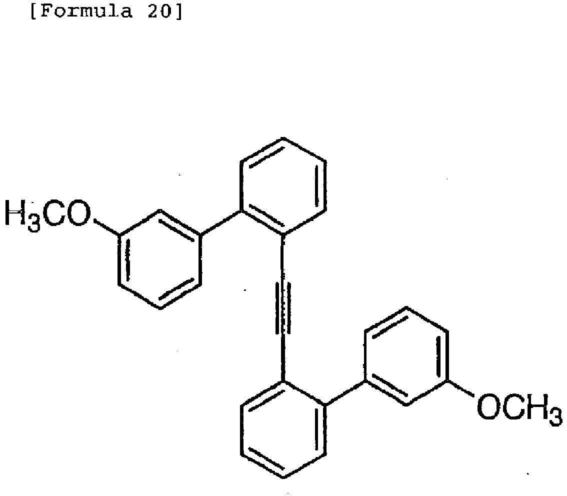

### Intermediate B

Into a 500 ml four-necked flask whose inside was replaced with a nitrogen gas, 13.3 g of the intermediate A, 15 g of 3-methoxyphenylboronic acid, 1.93 g of [1,1'-bis(diphenylphosphino)ferrocene]palladium(II)dichloride, 42 g of potassium phosphate, and 200 ml of DMF were charged, and the resulting mixture was reacted at 90°C for 8 hours. After the completion of the reaction, 300 ml of ethyl acetate was added and separation/washing of the reaction liquid was carried out three times with 300 ml of distilled water. The resulting organic layer was dried over magnesium sulfate and then the solvent was distilled off using a rotary evaporator. The resulting condensed product was purified by silica gel chromatography, and dispersed and washed with methanol and then the crystal was vacuum-dried to obtain 12.4 g of the desired product (light yellow crystal, yield 80.5%).

### (1-3) Synthesis of Intermediate C

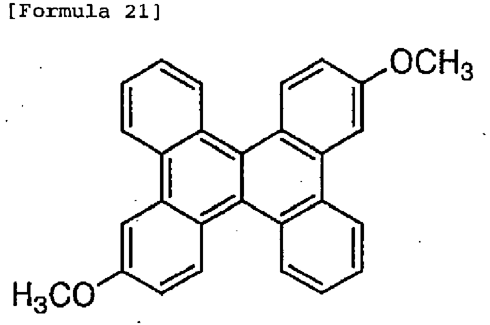

### Intermediate C

Into a 500 ml four-necked flask whose inside was replaced with a nitrogen gas, 12.4 g of the intermediate B and 150 ml of dehydrated dichloromethane were charged, and to this, 48 ml of a 1 M dichloromethane solution of antimony chloride was added dropwise over 20 minutes while stirring the resulting mixture. The resulting mixture was reacted for 2 hours as it is and 75 ml of methanol was added to terminate the reaction. Furthermore, 300 ml of dilute hydrochloric acid was added to perform separation/washing of the reaction liquid, and thereafter separation/washing by 300 ml of a saturated aqueous solution of ammonium chloride was carried out once and separation/washing by 300 ml of distilled water was carried out twice. The resulting organic layer was dried over magnesium sulfate and then the solvent was distilled off using a rotary evaporator, and the resulting viscous product was purified by silica gel chromatography. The resulting viscous product was recrystallized with ethyl acetate and then the crystal was vacuum-dried to obtain 5.1 g of the desired product (yellow crystal, yield 41.5%).

### (1-4) Synthesis of Intermediate D

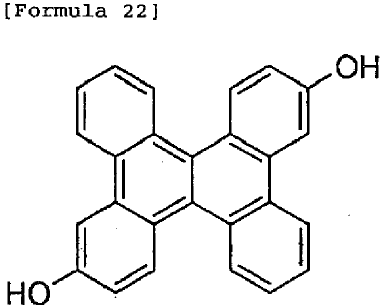

### Intermediate D

Into a 500 ml four-necked flask whose inside was replaced with a nitrogen gas, 5 g of the intermediate C and 150 ml of dehydrated dichloromethane were charged, and the resulting mixture was cooled to -60°C. 40 ml of a 1 M dichloromethane solution of boron tribromide was added dropwise to the solution over 20 minutes and the resulting mixture was returned to room temperature and reacted for 8 hours. After the completion of the reaction, 75 ml of concentrated ammonia water was added dropwise and the resulting mixture was stirred for 30 minutes. Thereafter, separation/washing was carried out three times with 200 ml of distilled water. The resulting organic layer was dried over magnesium sulfate and then the solvent was distilled off using a rotary evaporator, and the resulting solid was dispersed in a mixed liquid (mixing ratio 1 : 1) of acetone and methanol for washing and then the solid was vacuum-dried to obtain 4.1 g of the desired product (yellow crystal, yield 88.2%).

### (1-5) Synthesis of Intermediate E

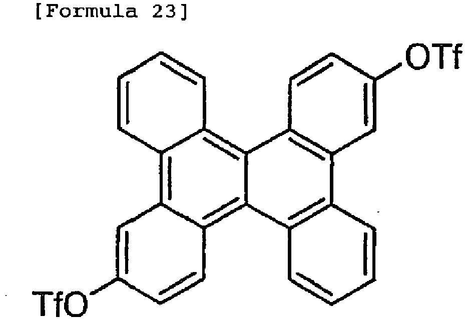

### Intermediate E

Into a 500 ml four-necked flask whose inside was replaced with a nitrogen gas, 4 g of the intermediate D, 150 ml of toluene, 7.1 g of potassium phosphate, and 80 ml of distilled water were charged, and the resulting mixture was heated to 50°C. To the mixture, 7.5 g of trifluoromethanesulfonic anhydride was added dropwise over 5 minutes while vigorously stirring to react the mixture for 1 hour. Since the proceeding of the reaction was inadequate, this operation was repeated another three times (total amount of trifluoromethanesulfonic anhydride was 30 g) to proceed the reaction adequately. After the completion of the reaction, the reaction liquid was cooled to room temperature and extracted with 200 ml of dichloromethane, and separation/washing of the reaction liquid was carried out four times with 300 ml of distilled water. The resulting organic layer was dried over magnesium sulfate and then the solvent was distilled off using a rotary evaporator, and the resulting solid was purified by silica gel chromatography and vacuum-dried to obtain 6.3 g of the desired product (white crystal, yield 90.3%).

### (1-6) Synthesis of Compound [29]

Into a 100 ml three-necked flask whose inside was replaced with a nitrogen gas, 1 g of the intermediate E, 1 g of p,p'-ditolylamine, 55.2 mg of bis(dibenzylideneacetone)palladium(0), 183 mg of 2-dicyclohexylphosphino-2',4',6'-triisopropylbiphenyl, 3.1 g of cesium carbonate, and 25 ml of xylene were charged, and the resulting mixture was reacted at 130°C for 6 hours. After the completion of the reaction, the reaction liquid was cooled to room temperature and filtrated and the solvent was distilled off using a rotary evaporator. The resulting solid was purified by silica gel chromatography and vacuum-dried to obtain 950 mg of the desired product (yellow crystal, yield 82.6%). This compound [29] was purified by sublimation at about 300°C at a pressure of 1.0 × 10⁻³ Pa using an oil diffusion pump and then used as a light emitting device material.
¹H-NMR(CDCl₃(d=ppm))2.35(s, 12H), 7.05-7.33(m, 18H), 7.45-7.60(m, 4H), 8.20-8.65(m, 8H).

### Synthesis Example 2 (Synthesis of Compound [37])

Synthesis was performed under the same charge ratio, reaction condition, and purification condition as in Synthesis Example 1, (1-6) Synthesis of Compound [29] except for using 1 g of the intermediate E and (4-tert-butylphenyl)phenylamine in place of p,p' -ditolylamine to obtain 970 mg of the desired product (yellow crystal, yield 78.2%) . This compound [37] was purified by sublimation at about 280°C at a pressure of 1.0 × 10⁻³ Pa using an oil diffusion pump and then used as a light emitting device material.
¹H-NMR(CDCl₃(d=ppm))1.33(s, 18H), 7.07-7.38(m, 20H),7.42-7.62(m, 4H) 8.23-8.65(m, 8H).

### Synthesis Example 3 (Synthesis of Compound [39])

Synthesis was performed under the same charge ratio, reaction condition, and purification condition as in Synthesis Example 1, (1-6) Synthesis of Compound [29] except for using 1 g of the intermediate E and bis(4-tert-butylphenyl)amine in place of p , p' -ditolylamine to obtain 1.15 g of the desired product (yellow crystal, yield 81.0%). This compound [39] was purified by sublimation at about 300°C at a pressure of 1.0 × 10⁻³ Pa using an oil diffusion pump and then used as a light emitting device material.
¹H-NMR(CDCl₃(d=ppm))1.33(s, 36H), 7.10-7.40(m, 18H), 7.45-7.64(m, 4H), 8.23-8.67(m, 8H).

### Synthesis Example 4 (Synthesis of Compound [40])

Synthesis was performed under the same charge ratio, reaction condition, and purification condition as in Synthesis Example 1, (1-6) Synthesis of Compound [29] except for using 1 g of the intermediate E and (3-tert-butylphenyl)phenylamine in place of p,p'-ditolylamine to obtain 980 mg of the desired product (yellow crystal, yield 79.0%). This compound [40] was purified by sublimation at about 300°C at a pressure of 1.0 × 10⁻³ Pa using an oil diffusion pump and then used as a light emitting device material.
¹H-NMR(CDCl₃(d=ppm))1.25(s, 18H), 6.96-7.45(m, 20H), 7.48-7.67(m, 4H), 8.27-8.74(m, 8H).

### Example 1

A light emitting device using the compound [29] was prepared as follows. An ITO conductive film was formed in a thickness of 150 nm and a size of 30 × 13 mm on a central part of a glass substrate (produced by Asahi Glass Co., Ltd., 15 Ω/square, electron beam evaporation product) having a size of 30 × 40 mm to form an anode. The substrate provided with the anode was cleaned for 15 minutes through ultrasonic action by "SEMICOCLEAN (registered trade mark) 56" (manufactured by Furuuchi Chemical Corporation) and then cleaned with ultrapure water. Subsequently, the substrate was cleaned for 15 minutes in isopropyl alcohol through ultrasonic action, immersed for 15 minutes in hot methanol and dried. The substrate was treated in an atmosphere of UV-ozone for 1 hour immediately before preparing the device and then placed in a vacuum evaporation system, and the system was evacuated until the degree of vacuum in the system became 5 × 10⁻⁵ Pa or less. Copper phthalocyanine as a hole injection material was first deposited in a thickness of 10 nm and 4,4'-bis(N-(1-naphthyl)-N-phenylamino)biphenyl as a hole transporting material was deposited in a thickness of 50 nm on the ITO film of the substrate by a resistance heating evaporation method. Next, H-1 represented by the following formula was deposited as a host material of the emissive layer and the compound [29] was deposited in a thickness of 35 nm so as to have a doping concentration of 5% by weight as a dopant material. Next, tris(8-quinolinolato)aluminum (Alq₃) as an electron transporting material was laminated in a thickness of 20 nm. Lithium fluoride was deposited in a thickness of 0.5 nm and aluminum was deposited in a thickness of 1000 nm on the organic layer thus formed to form a cathode to prepare a 5 mm square device. The film thickness referred to herein is an indicated value of a crystal oscillation type film thickness monitor. When the light emitting device was driven at a direct current of 10 mA/cm², emission of high efficiency with a luminance efficiency of 3.2 lm/W and pure blue emission with CIE chromaticity coordinates of (0.14, 0.16) were achieved. The initial luminance of this device was set at 1000 cd/m² and the device was continuously driven with a direct current, and consequently the half-life of luminance was 2500 hours, indicating a long life.

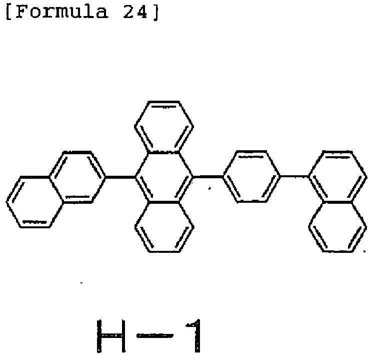

### Examples 2 to 36

Light emitting devices were prepared in the same manner as in Example 1 except for using compounds shown in Table 1, respectively, as a dopant material, a host material of an emissive layer and an electron transporting material. With respect to these light emitting devices, the results of the luminance efficiency at the time of driving the device at a direct current of 10 mA/cm², CIE chromaticity coordinates, and the half-life of luminance at the time when the initial luminance was set at 1000 cd/m² and the device was driven at a direct current are shown in Table 1. In Table 1, H-2, H-3, H-4, H-5, H-6, H-7, H-8 and H-9 represent compounds represented by the following formulas.

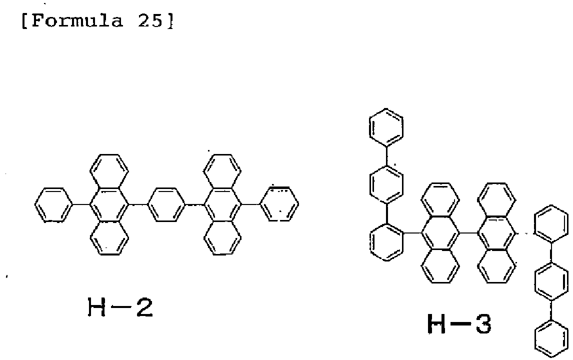

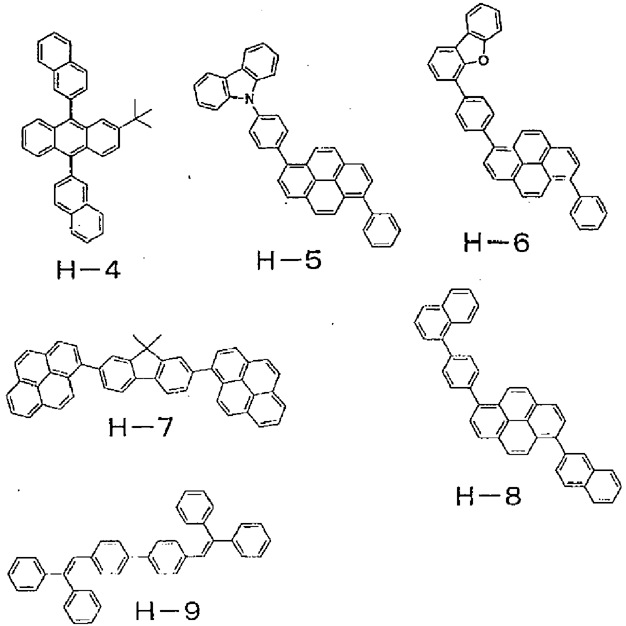

### Comparative Examples 1 to 20

Light emitting devices were prepared in the same manner as in Example 1 except for using compounds shown in Table 2, respectively, as a dopant material, a host material and an electron transporting material. With respect to these light emitting devices, the results of the luminance efficiency at the time of driving the device at a direct current of 10 mA/cm², CIE chromaticity coordinates, and the half-life of luminance at the time when the initial luminance was set at 1000 cd/m² and the device was driven at a direct current are shown in Table 2. However, the half-life of luminance of the device of Comparative Example 10 was not measured since the device exhibited poor color purity. In Table 2, D-1, D-2, D-3 and D-4 are compounds represented by the following formulas.

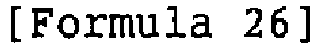

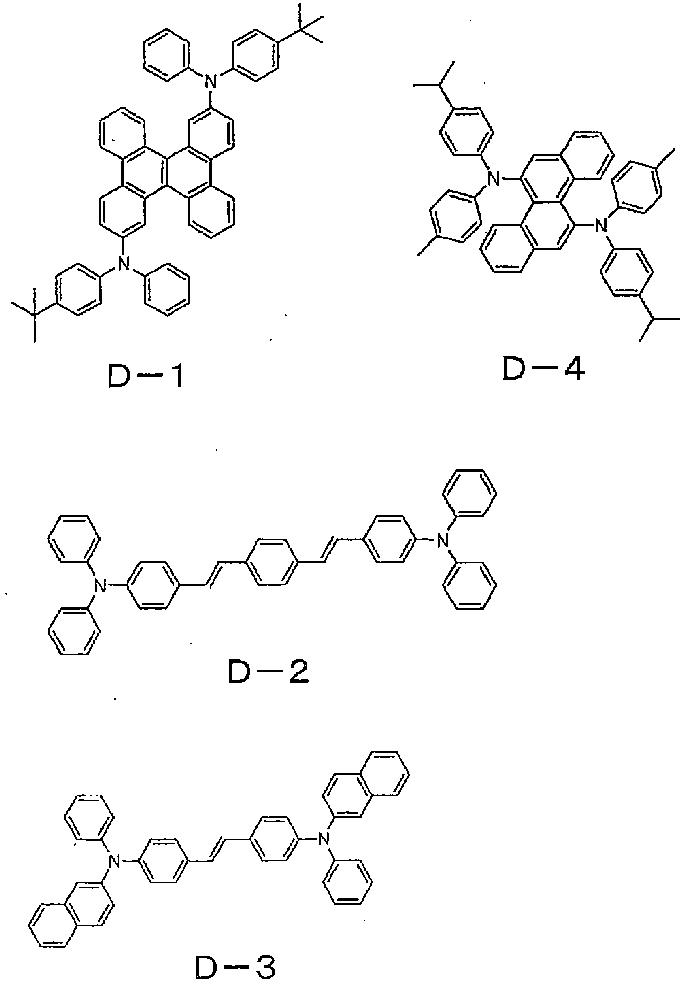

From the comparisons with Examples 1 to 36, it is evident that the compound represented by the general formula (1) exhibits excellent performance in all of luminance efficiency, color purity and a life as a dopant material. That is, effects of the invention of claims 1 to 5 of the present invention are shown.

### Examples 37 to 54

Light emitting devices were prepared in the same manner as in Example 1 except for using compounds shown in Table 3, respectively, as a dopant material, a host material and an electron transporting material. With respect to these light emitting devices, the results of the luminance efficiency at the time of driving the device at a direct current of 10 mA/cm², CIE chromaticity coordinates, and the half-life of luminance at the time when the initial luminance was set at 1000 cd/m² and the device was driven at a direct current are shown in Table 3. In Table 3, E-1, E-2, E-3, E-4 and E-5 are compounds represented by the following formulas.

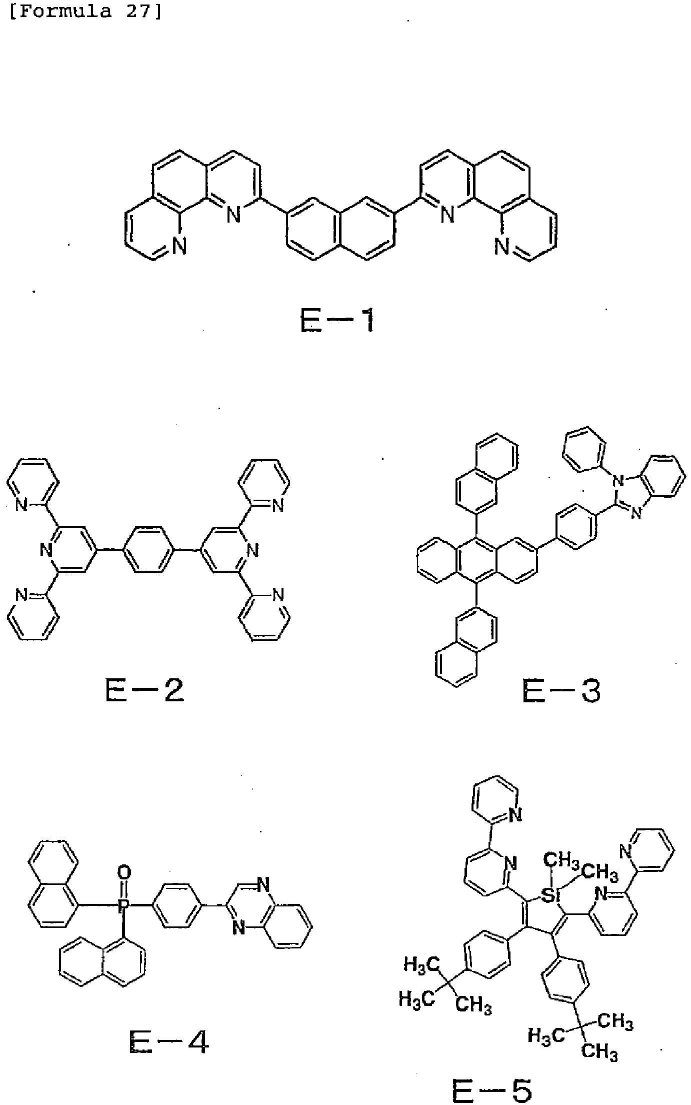

### Comparative Examples 21 to 38

Light emitting devices were prepared in the same manner as in Example 1 except for using compounds shown in Table 3, respectively, as a dopant material, a host material and an electron transporting material. With respect to these light emitting devices, the results of the luminance efficiency at the time of driving the device at a direct current of 10 mA/cm², CIE chromaticity coordinates, and the half-life of luminance at the time when the initial luminance was set at 1000 cd/m² and the device was driven at a direct current are shown in Table 3.

### Example 55

A light emitting device using the compound [29] as a hole injection material was prepared as follows. An ITO conductive film was formed in a thickness of 150 nm and a size of 30 x 13 mm on a central part of a glass substrate (produced by Asahi Glass Co., Ltd. , 15 Ω/square, electron beam evaporation product) having a size of 30 x 40 mm to form an anode. The substrate provided with the anode was cleaned for 15 minutes through ultrasonic action by "SEMICOCLEAN (registered trade mark) 56" (manufactured by Furuuchi Chemical Corporation) and then cleaned with ultrapure water. Subsequently, the substrate was cleaned for 15 minutes in isopropyl alcohol through ultrasonic action, immersed for 15 minutes in hot methanol and dried. The substrate was treated in an atmosphere of UV-ozone for 1 hour immediately before preparing the device and then placed in a vacuum evaporation system, and the system was evacuated until the degree of vacuum in the system became 5 × 10⁻⁵ Pa or less. The compound [29] as a hole injection material was first deposited in a thickness of 40 nm and 4,4'-bis(N-(1-naphthyl)-N-phenylamino)biphenyl as a hole transporting material was deposited in a thickness of 10 nm on the ITO film of the substrate by a resistance heating evaporation method. Next, H-1 was deposited as a host material of the emissive layer and D-4 was deposited in a thickness of 35 nm so as to have a doping concentration of 5% by weight as a dopant material. Next, tris(8-quinolinolato)aluminum (Alq₃) as an electron transporting material was laminated in a thickness of 20 nm. Lithium fluoride was deposited in a thickness of 0.5 nm and aluminum was deposited in a thickness of 1000 nm on the organic layer thus formed to form a cathode to prepare a 5 mm square device. The film thickness referred to herein is an indicated value of a crystal oscillation type film thickness monitor. When the light emitting device was driven at a direct current of 10 mA/cm², emission of high efficiency with a luminance efficiency of 3.5 lm/W and pure blue emission with CIE chromaticity coordinates of (0.14, 0.14) were achieved. The initial luminance of this device was set at 1000 cd/m² and the device was continuously driven with a direct current, and consequently the half-life of luminance was 2500 hours, indicating a long life.

### Examples 56 to 69

Light emitting devices were prepared in the same manner as in Example 55 except for using compounds shown in Table 4, respectively, as a hole injection material, a host material and a dopant material. With respect to these light emitting devices, the results of the luminance efficiency at the time of driving the device at a direct current of 10 mA/cm², CIE chromaticity coordinates, and a half-life of luminance at the time when the initial luminance was set at 1000 cd/m² and the device was driven at a direct current are shown in Table 4.

### Comparative Examples 39 to 42

Light emitting devices were prepared in the same manner as in Example 55 except for using compounds shown in Table 4, respectively, as a hole injection material, a host material and a dopant material. With respect to these light emitting devices , the results of the luminance efficiency at the time of driving the device at a direct current of 10 mA/cm², CIE chromaticity coordinates, and the half-life of luminance at the time when the initial luminance was set at 1000 cd/m² and the device was driven at a direct current are shown in Table 4. In Table 4, HI-1 and HI-2 are compounds represented by the following formulas.

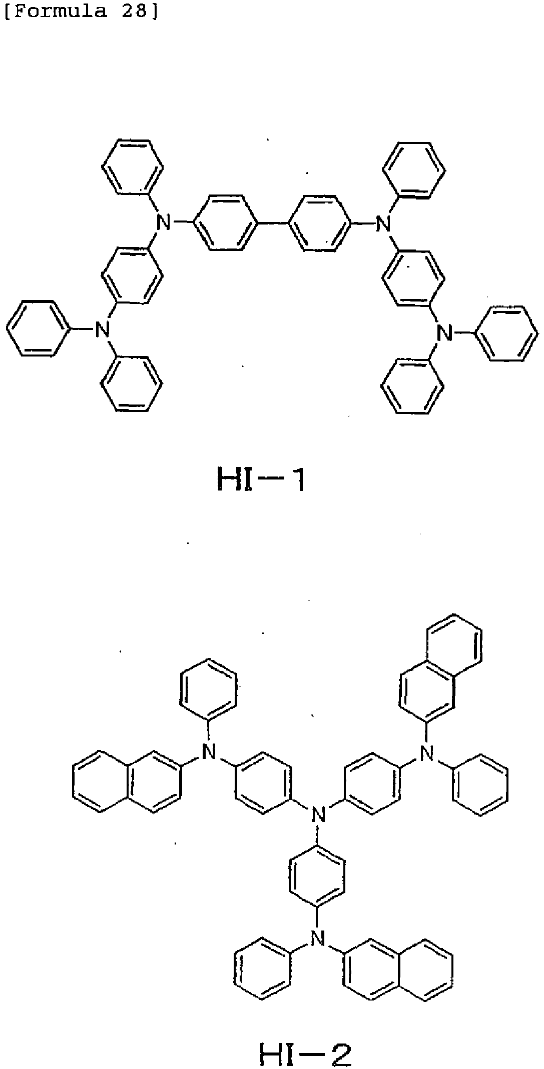

From the comparisons with Examples 55 to 69, it is evident that the compound represented by the general formula (3) exhibits excellent performance of improving the life as a hole injection material. That is, effects of the invention of claims 6 to 8 of the present invention are shown. Further, from the comparison with Example 5, it is evident that a compound, which is represented by the general formula (1) and does not correspond to the general formula (3), like the compound [69], exhibits excellent performance as a dopant material. That is, effects of the invention of claims 1 to 5 of the present invention are shown.

### Examples 70 to 87

Light emitting devices were prepared in the same manner as in Example 55 except for using compounds shown in Table 5, respectively, as a hole injection material, a host material and a dopant material. With respect to these light emitting devices, the results of the luminance efficiency at the time of driving the device at a direct current of 10 mA/cm², CIE chromaticity coordinates, and the half-life of luminance at the time when the initial luminance was set at 1000 cd/m² and the device was driven at a direct current are shown in Table 5.

### Comparative Examples 43 to 69

Light emitting devices were prepared in the same manner as in Example 55 except for using compounds shown in Table 6, respectively, as a hole injection material, a host material and a dopant material. With respect to these light emitting devices, the results of the luminance efficiency at the time of driving the device at a direct current of 10 mA/cm², CIE chromaticity coordinates, and the half-life of luminance at the time when the initial luminance was set at 1000 cd/m² and the device was driven at a direct current are shown in Table 6.

### Examples 88 to 99

Light emitting devices were prepared in the same manner as in Example 55 except for using compounds shown in Table 7, respectively, as a hole injection material, a host material, a dopant material and an electron transporting material. With respect to these light emitting devices, the results of the luminance efficiency at the time of driving the device at a direct current of 10 mA/cm², CIE chromaticity coordinates, and the half-life of luminance at the time when the initial luminance was set at 1000 cd/m² and the device was driven at a direct current are shown in Table 7.

### Comparative Examples 70 to 87

Light emitting devices were prepared in the same manner as in Example 55 except for using compounds shown in Table 7, respectively, as a hole injection material, a host material, a dopant material and an electron transporting material. With respect to these light emitting devices, the results of the luminance efficiency at the time of driving the device at a direct current of 10 mA/cm², CIE chromaticity coordinates, and the half-life of luminance at the time when the initial luminance was set at 1000 cd/m² and the device was driven at a direct current are shown in Table 7.

**Table 1**

| | Dopant | Host | Electron transporting layer | luminance efficiency (Im/W) | CIE chromaticity coordinates (x,y) | half-life of luminance (hr) |
|---|---|---|---|---|---|---|
| Example 1 | compound[29] | H-1 | Alq₃ | 3.2 | (0.14,0.16) | 2500 |
| Example 2 | compound[37] | H-1 | Alq₃ | 3.5 | (0.14,0.14) | 4500 |
| Example 3 | compound[39] | H-1 | Alq₃ | 3.7 | (0.15,0.15) | 4000 |
| Example 4 | compound[40] | H-1 | Alq₃ | 3.5 | (0.14,0.14) | 5500 |
| Example 5 | compound[69] | H-1 | Alq₃ | 3.5 | (0.14,0.20) | 2000 |
| Example 6 | compound[41] | H-1 | Alq₃ | 3.6 | (0.14,0.15) | 3700 |
| Example 7 | compound[6] | H-1 | Alq₃ | 3.7 | (0.14,0.17) | 2000 |
| Example 8 | compound[34] | H-1 | Alq₃ | 3.5 | (0.14,0.15) | 4500 |
| Example 9 | compound[63] | H-1 | Alq₃ | 3.7 | (0.14,0.15) | 4400 |
| Example 10 | compound[1] | H-1 | Alq₃ | 3.8 | (0.14,0.17) | 3000 |
| Example 11 | compound[2] | H-1 | Alq₃ | 3.9 | (0.14,0.19) | 3100 |
| Example 12 | compound[18] | H-1 | Alq₃ | 3.7 | (0.14,0.17) | 3500 |
| Example 13 | compound[37] | H-2 | Alq₃ | 3.6 | (0.14,0.14) | 4600 |
| Example 14 | compound[39] | H-2 | Alq₃ | 3.7 | (0.15,0.15) | 4700 |
| Example 15 | compound[40] | H-2 | Alq₃ | 3.5 | (0.14,0.14) | 5200 |
| Example 16 | compound[37] | H-3 | Alq₃ | 3.6 | (0.14,0.14) | 3900 |
| Example 17 | compound[39] | H-3 | Alq₃ | 3.4 | (0.15,0.15) | 4200 |
| Example 18 | compound[40] | H-3 | Alq₃ | 3.5 | (0.14,0.14) | 4400 |
| Example 19 | compound[37] | H-4 | Alq₃ | 3.3 | (0.14,0.14) | 3600 |
| Example 20 | compound[39] | H-4 | Alq₃ | 3.5 | (0.15,0.15) | 3800 |
| Example 21 | compound[40] | H-4 | Alq₃ | 3.5 | (0.14,0.14) | 4200 |
| Example 22 | compound[37] | H-5 | Alq₃ | 3.5 | (0.14,0.15) | 2800 |
| Example 23 | compound[39] | H-5 | Alq₃ | 3.7 | (0.14,0.15) | 2800 |
| Example 24 | compound[40] | H-5 | Alq₃ | 3.4 | (0.14,0.14) | 3000 |
| Example 25 | compound[37] | H-6 | Alq₃ | 3.7 | (0.14,0.15) | 3200 |
| Example 26 | compound[39] | H-6 | Alq₃ | 3.8 | (0.14,0.15) | 3500 |
| Example 27 | compound[40] | H-6 | Alq₃ | 3.7 | (0.14,0.14) | 3700 |
| Example 28 | compound[37] | H-7 | Alq₃ | 3.6 | (0.14,0.15) | 3300 |
| Example 29 | compound[39] | H-7 | Alq₃ | 3.8 | (0.14,0.15) | 3500 |
| Example 30 | compound[40] | H-7 | Alq₃ | 3.6 | (0.14,0.14) | 3800 |
| Example 31 | compound[37] | H-8 | Alq₃ | 3.8 | (0.14,0.15) | 3200 |
| Example 32 | compound[39] | H-8 | Alq₃ | 3.7 | (0.14,0.15) | 3300 |
| Example 33 | compound[40] | H-8 | Alq₃ | 3.7 | (0.14,0.14) | 3100 |
| Example 34 | compound[37] | H-9 | Alq₃ | 3.5 | (0.14,0.14) | 1800 |
| Example 35 | compound[39] | H-9 | Alq₃ | 3.5 | (0.14,0.15) | 2000 |
| Example 36 | compound[40] | H-9 | Alq₃ | 3.6 | (0.14,0.14) | 2100 |

**Table 2**

| | Dopant | Host | Electron transporting layer | Luminance efficiency (Im/W) | CIE chromaticity coordinates (x,y) | half-life of Luminance (hr) |
|---|---|---|---|---|---|---|
| Comparative example 1 | D-1 | H-1 | Alq₃ | 2.9 | (0.15,0.18) | 900 |
| Comparative example 2 | D-1 | H-2 | Alq₃ | 2.8 | (0.15,0.18) | 900 |
| Comparative example 3 | D-1 | H-3 | Alq₃ | 2.6 | (0.15,0.19) | 700 |
| Comparative example 4 | D-1 | H-4 | Alq₃ | 2.6 | (0.14,0.18) | 600 |
| Comparative example 5 | D-1 | H-5 | Alq₃ | 2.8 | (0.15,0.19) | 800 |
| Comparative example 6 | D-1 | H-6 | Alq₃ | 2.7 | (0.14,0.18) | 700 |
| Comparative example 7 | D-1 | H-7 | Alq₃ | 2.6 | (0.14,0.18) | 800 |
| Comparative example 8 | D-1 | H-8 | Alq₃ | 2.8 | (0.14,0.18) | 800 |
| Comparative example 9 | D-1 | H-9 | Alq₃ | 2.7 | (0.14,0.19) | 500 |
| Comparative example 10 | D-2 | H-1 | Alq₃ | 4.4 | (0.16,0.27) | - |
| Comparative example 11 | D-3 | H-1 | Alq₃ | 2.7 | (0.15,0.15) | 800 |
| Comparative example 12 | D-4 | H-1 | Alq₃ | 2.5 | (0.14,0.14) | 1000 |
| Comparative example 13 | D-4 | H-2 | Alq₃ | 2.4 | (0.14,0.14) | 800 |
| Comparative example 14 | D-4 | H-3 | Alq₃ | 2.5 | (0.14,0.14) | 600 |
| Comparative example 15 | D-4 | H-4 | Alq₃ | 2.6 | (0.14,0.14) | 700 |
| Comparative example 16 | D-4 | H-5 | Alq₃ | 2.5 | (0.14,0.15) | 600 |
| Comparative example 17 | D-4 | H-6 | Alq₃ | 2.7 | (0.14,0.15) | 800 |
| Comparative example 18 | D-4 | H-7 | Alq₃ | 2.7 | (0.14,0.15) | 700 |
| Comparative example 19 | D-4 | H-8 | Alq₃ | 2.6 | (0.14,0.15) | 600 |
| Comparative example 20 | D-4 | H-9 | Alq₃ | 2.7 | (0.14,0,14) | 400 |

**Table 3**

| | Dopant | Host | Electron transporting layer | Luminance efficiency (Im/W) | CIE chromaticity coordinates (x,y) | half-life of Luminance (hr) |
|---|---|---|---|---|---|---|
| Example 37 | compound[37] | H-1 | E-1 | 5.2 | (0.14,0.15) | 2500 |
| Example 38 | compound[37] | H-5 | E-1 | 5.9 | (0.14,0.15) | 3000 |
| Example 39 | compound[37] | H-5 | E-2 | 5.7 | (0.14,0.14) | 4300 |
| Example 40 | compound[37] | H-5 | E-3 | 5.3 | (0.14,0.14) | 3000 |
| Example 41 | compound[37] | H-5 | E-4 | 5.8 | (0.14,0.14) | 3300 |
| Example 42 | compound[37] | H-5 | E-5 | 5.4 | (0.14,0.14) | 2200 |
| Example 43 | compound[39] | H-1 | E-1 | 5.4 | (0.14,0.15) | 2300 |
| Example 44 | compound[39] | H-5 | E-1 | 6 | (0.14,0.15) | 2800 |
| Example 45 | compound[39] | H-5 | E-2 | 5.8 | (0.14,0.15) | 3900 |
| Example 46 | compound[39] | H-5 | E-3 | 5.3 | (0.15,0.15) | 3000 |
| Example 47 | compound[39] | H-5 | E-4 | 5.6 | (0.15,0.15) | 3200 |
| Example 48 | compound[39] | H-5 | E-5 | 5.4 | (0.15,0.15) | 2400 |
| Example 49 | compound[40] | H-1 | E-1 | 5.3 | (0.14,0.14) | 2900 |
| Example 50 | compound[40] | H-5 | E-1 | 6 | (0.14,0.14) | 3600 |
| Example 51 | compound[40] | H-5 | E-2 | 5.6 | (0.14,0.14) | 4500 |
| Example 52 | compound[40] | H-5 | E-3 | 5.2 | (0.14,0.14) | 3000 |
| Example 53 | compound[40] | H-5 | E-4 | 5.7 | (0.14,0.14) | 3500 |
| Example 54 | compound[40] | H-5 | E-5 | 5.3 | (0.14,0.14) | 2200 |
| Comparative example 21 | D-1 | H-1 | E-1 | 4 | (0.14,0.18) | 400 |
| Comparative example 22 | D-1 | H-5 | E-1 | 4.4 | (0.14,0.19) | 800 |
| Comparative example 23 | D-1 | H-5 | E-2 | 4 | (0.15,0.18) | 1100 |
| Comparative example 24 | D-1 | H-5 | E-3 | 3.7 | (0.14,0.18) | 800 |
| Comparative example 25 | D-1 | H-5 | E-4 | 3.9 | (0.15,0.19) | 800 |
| Comparative example 26 | D-1 | H-5 | E-5 | 3.5 | (0.15,0.19) | 600 |
| Comparative example 27 | D-3 | H-1 | E-1 | 3.9 | (0.15,0.15) | 200 |
| Comparative example 28 | D-3 | H-5 | E-1 | 4.4 | (0.15,0.15) | 600 |
| Comparative example 29 | D-3 | H-5 | E-2 | 4 | (0.15,0.15) | 900 |
| Comparative example 30 | D-3 | H-5 | E-3 | 3.7 | (0.15,0.15) | 500 |
| Comparative example 31 | D-3 | H-5 | E-4 | 3.9 | (0.15,0.15) | 800 |
| Comparative example 32 | D-3 | H-5 | E-5 | 3.6 | (0.15,0.15) | 300 |
| Comparative example 33 | D-4 | H-1 | E-1 | 3.8 | (0.14,0.14) | 500 |
| Comparative example 34 | D-4 | H-5 | E-1 | 4.2 | (0.14,0.14) | 1000 |
| Comparative example 35 | D-4 | H-5 | E-2 | 3.7 | (0.14,0.14) | 1300 |
| Comparative example 36 | D-4 | H-5 | E-3 | 3.5 | (0.14,0.14) | 800 |
| Comparative example 37 | D-4 | H-5 | E-4 | 4 | (0.14,0.14) | 1000 |
| Comparative example 38 | D-4 | H-5 | E-5 | 3.5 | (0.14,0.14) | 600 |

**Table 4**

| | Hole injection layer | Host | Dopant | luminance efficiency (Im/W) | CIE chromaticity coordinates (x,y) | half-life of luminance (hr) |
|---|---|---|---|---|---|---|
| Example 55 | compound[29] | H-1 | D-4 | 3.5 | (0.14,0.14) | 2500 |
| Example 56 | compound[37] | H-1 | D-4 | 3.6 | (0.14,0.14) | 2400 |
| Example 57 | compound[32] | H-1 | D-4 | 3.6 | (0.14,0.14) | 2200 |
| Example 58 | compound[39] | H-1 | D-4 | 3.7 | (0.14,0.14) | 2000 |
| Example 59 | compound[40] | H-1 | D-4 | 3.4 | (0.14,0.14) | 2300 |
| Example 60 | compound[4] | H-1 | D-4 | 3.4 | (0.14,0.15) | 2500 |
| Example 61 | compound[6] | H-1 | D-4 | 3.4 | (0.14,0.15) | 2600 |
| Example 62 | compound[25] | H-1 | D-4 | 3.6 | (0.14,0.14) | 2100 |
| Example 63 | compound[30] | H-1 | D-4 | 3.6 | (0.14,0.14) | 2500 |
| Example 64 | compound[27] | H-1 | D-4 | 3.7 | (0.14,0.14) | 2400 |
| Example 65 | compound[33] | H-1 | D-4 | 3.5 | (0.14,0.14) | 2500 |
| Example 66 | compound[1] | H-1 | D-4 | 3.5 | (0.14,0.14) | 2300 |
| Example 67 | compound[2] | H-1 | D-4 | 3.4 | (0.14,0.14) | 2300 |
| Example 68 | compound[18] | H-1 | D-4 | 3.6 | (0.14,0.14) | 2500 |
| Example 69 | compound[41] | H-1 | D-4 | 3.6 | (0.14,0.14) | 2100 |
| Comparative example 39 | HI-1 | H-1 | D-4 | 3.3 | (0.15,0.14) | 1000 |
| Comparative example 40 | HI-2 | H-1 | D-4 | 2.7 | (0.14,0.14) | 800 |
| Comparative example 41 | compound[69] | H-1 | D-4 | 2.8 | (0.14,0.14) | 800 |
| Comparative example 42 | D-1 | H-1 | D-4 | 2.7 | (0.14,0.14) | 700 |

**Table 5**

| | Hole injection layer | Host | Dopant | Luminance efficiency (Im/W) | CIE chromaticity coordinates (x,y) | half-life of Luminance (hr) |
|---|---|---|---|---|---|---|
| Example 70 | compouud[29] | H-1 | compound[40] | 4 | (0.14,0.14) | 9300 |
| Example 71 | compound[29] | H-2 | compound[40] | 3.9 | (0.14,0.14) | 9000 |
| Example 72 | compound[29] | H-3 | compound[40] | 4.1 | (0.14,0.14) | 8200 |
| Example 73 | compound[29] | H-4 | compound[40] | 4.1 | (0.14,0.14) | 8700 |
| Example 74 | compound[29] | H-5 | compound[40] | 3.8 | (0.14,0.15) | 8800 |
| Example 75 | compound[29] | H-6 | compound[40] | 3.9 | (0.14,0.14) | 8500 |
| Example 76 | compound[29] | H-7 | compound[40] | 4.1 | (0.14,0.14) | 8500 |
| Example 77 | compound[29] | H-8 | compound[40] | 4 | (0.14,0.14) | 8400 |
| Example 78 | compound[29] | H-9 | compound[40] | 3.8 | (0.14,0.14) | 6500 |
| Example 79 | compound[37] | H-1 | compound[40] | 4.1 | (0.14,0.14) | 9500 |
| Example 80 | compound[37] | H-2 | compound[40] | 4.2 | (0.14,0.14) | 9000 |
| Example 81 | compound[37] | H-3 | compound[40] | 4.2 | (0.14,0.14) | 8200 |
| Example 82 | compound[37] | H-4 | compound[40] | 4 | (0.14,0.14) | 8900 |
| Example 83 | compound[37] | H-5 | compound[40] | 3.9 | (0.14,0.14) | 8500 |
| Example 84 | compound[37] | H-6 | compound[40] | 3.9 | (0.14,0.15) | 8700 |
| Example 85 | compound[37] | H-7 | compound[40] | 4.2 | (0.14,0.14) | 8700 |
| Example 86 | compound[37] | H-8 | compound[40] | 4 | (0.14,0.14) | 8400 |
| Example 87 | compound[37] | H-9 | compound[40] | 3.9 | (0.14,0.14) | 6200 |

**Table 6**

| | Hole injection layer | Host | Dopant | Luminance efficiency (Im/W) | CIE chromaticity coordinates (x,y) | half-life of luminance (hr) |
|---|---|---|---|---|---|---|
| Comparative example 43 | HI-1 | H-1 | compound[40] | 3.8 | (0.14,0.14) | 6200 |
| Comparative example 44 | HI-1 | H-2 | compound[40] | 3.9 | (0.14,0.14) | 6000 |
| Comparative example 45 | HI-1 | H-3 | compound[40] | 4 | (0.14,0.14) | 5500 |
| Comparative example 46 | HI-1 | H-4 | compound[40] | 4.1 | (0.14,0.14) | 6000 |
| Comparative example 47 | HI-1 | H-5 | compound[40] | 3.9 | (0.14,0,14) | 5300 |
| Comparative example 48 | HI-1 | H-6 | compound[40] | 3.8 | (0.14,0,14) | 5100 |
| Comparative example 49 | HI-1 | H-7 | compound[40] | 4.1 | (0.14,0,14) | 5000 |
| Comparative example 50 | HI-1 | H-8 | compound[40] | 4.1 | (0.14,0,14) | 5500 |
| Comparative example 51 | HI-1 | H-9 | compound[40] | 3.9 | (0.14,0,14) | 4000 |
| Comparative example 52 | compound[69] | H-1 | compound[40] | 3.8 | (0.14,0.14) | 5400 |
| Comparative example 53 | compound[69] | H-2 | compound[40] | 3.7 | (0.14,0.14) | 5300 |
| Comparative example 54 | compound[69] | H-3 | compound[40] | 3.6 | (0.14,0.14) | 4800 |
| Comparative example 55 | compound[69] | H-4 | compound[40] | 3.8 | (0.14,0.14) | 5200 |
| Comparative example 56 | compound[69] | H-5 | compound[40] | 3.9 | (0.14,0.14) | 5500 |
| Comparative example 57 | compound[69] | H-6 | compound[40] | 3.9 | (0.14,0.14) | 5400 |
| Comparative example 58 | compound[69] | H-7 | compound[40] | 3.8 | (0.14,0.14) | 5100 |
| Comparative example 59 | compound[69] | H-8 | compound[40] | 3.8 | (0.14,0.14) | 5100 |
| Comparative example 60 | compound[69] | H-9 | compound[40] | 3.7 | (0.14,0.14) | 4300 |
| Comparative example 61 | D-1 | H-1 | compound[40] | 3.9 | (0.14,0.14) | 5700 |
| Comparative example 62 | D-1 | H-2 | compound[40] | 3.7 | (0.14,0.14) | 5500 |
| Comparative example 63 | D-1 | H-3 | compound[40] | 3.6 | (0.14,0.14) | 4600 |
| Comparative example 64 | D-1 | H-4 | compound[40] | 3.7 | (0.14,0.14) | 5300 |
| Comparative example 65 | D-1 | H-5 | compound[40] | 3.7 | (0.14,0.14) | 5300 |
| Comparative example 66 | D-1 | H-6 | compound[40] | 4 | (0.14,0.14) | 5500 |
| Comparative example 67 | D-1 | H-7 | compound[40] | 3.8 | (0.14,0.14) | 4900 |
| Comparative example 68 | D-1 | H-8 | compound[40] | 3.8 | (0.14,0.14) | 5000 |
| Comparative example 69 | D-1 | H-9 | compound[40] | 3.7 | (0.14,0.14) | 3800 |

**Table 7**

| | Hole injection layer | Host | Dopant | Electron transporting layer | Luminance efficiency (Im/W) | chromaticity coordinates (x,y) | half-life of Luminance (hr) |
|---|---|---|---|---|---|---|---|
| Example 88 | compound[29] | H-1 | compound[40] | E-1 | 5.7 | (0.14,0.14) | 3400 |
| Example 89 | compound[29] | H-5 | compound[40] | E-1 | 6.2 | (0.14,0.14) | 5200 |
| Example 90 | compound[29] | H-5 | compound[40] | E-2 | 5.7 | (0.14,0.14) | 5500 |
| Example 91 | compound[29] | H-5 | compound[40] | E-3 | 5.8 | (0.14,0.14) | 4700 |
| Example 92 | compound[29] | H-5 | compound[40] | E-4 | 6 | (0.14,0.14) | 5000 |
| Example 93 | compound[29] | H-5 | compound[40] | E-5 | 5.5 | (0.14,0.14) | 4100 |
| Example 94 | compound[37] | H-1 | compound[40] | E-1 | 5.6 | (0.14,0.14) | 3300 |
| Example 95 | compound[37] | H-5 | compound[40] | E-1 | 6 | (0.14,0.14) | 5100 |
| Example 96 | compound[37] | H-5 | compound[40] | E-2 | 5.6 | (0.14,0.14) | 5800 |
| Example 97 | compound[37] | H-5 | compound[40] | E-3 | 5.5 | (0.14,0.14) | 4500 |
| Example 98 | compound[37] | H-5 | compound[40] | E-4 | 5.8 | (0.14,0.14) | 4800 |
| Example 99 | compound[37] | H-5 | compound[40] | E-5 | 5.3 | (0.14,0.14) | 3900 |
| Comparative example 70 | HI-1 | H-1 | compound[40] | E-1 | 5.6 | (0.14,0.14) | 1000 |
| Comparative example 71 | HI-1 | H-5 | compound[40] | E-1 | 6 | (0.14,0.14) | 1300 |
| Comparative example 72 | HI-1 | H-5 | compound[40] | E-2 | 5.6 | (0.14,0.14) | 1600 |
| Comparati ve example 73 | HI-1 | H-5 | compound[40] | E-3 | 5.6 | (0.14,0.14) | 1400 |
| Comparative example 74 | HI-1 | H-5 | compound[40] | E-4 | 5.8 | (0.14,0.14) | 1300 |
| Comparative example 75 | HI-1 | H-5 | compound[40] | E-5 | 5.3 | (0.14,0.14) | 1000 |
| Comparative example 76 | compound[69] | H-1 | compound[40] | E-1 | 5.6 | (0.14,0.14) | 1200 |
| Comparative example 77 | compound[69] | H-5 | compound[40] | E-1 | 5.9 | (0.14,0.14) | 1500 |
| Comparative example 78 | compound[69] | H-5 | compound[40] | E-2 | 5.5 | (0.14,0.14) | 1300 |
| Comparative example 79 | compound[69] | H-5 | compound[40] | E-3 | 5.7 | (0.14,0.14) | 1200 |
| Comparative example 80 | compound[69] | H-5 | compound[40] | E-4 | 5.8 | (0.14,0.14) | 1100 |
| Comparative example 81 | compound[69] | H-5 | compound[40] | E-5 | 5.2 | (0.14,0.14) | 900 |
| Comparati ve example 82 | D-1 | H-1 | compound[40] | E-1 | 5.8 | (0.14,0.14) | 1200 |
| Comparative example 83 | D-1 | H-5 | compound[40] | E-1 | 6.1 | (0.14,0.14) | 1600 |
| Comparative example 84 | D-1 | H-5 | compound[40] | E-2 | 5.7 | (0.14,0.14) | 1500 |
| Comparative example 85 | D-1 | H-5 | compound[40] | E-3 | 5.5 | (0.14,0.14) | 1300 |
| Comparative example 86 | D-1 | H-5 | compound[40] | E-4 | 5.8 | (0.14,0.14) | 1300 |
| Comparative example 87 | D-1 | H-5 | compound[40] | E-5 | 5.4 | (0.14,0.14) | 1100 |

The light emitting device material of the present invention can be used for light emitting devices and can provide a light emitting device material which is excellent in thin film stability. The light emitting device of the present invention can be used for the fields of display devices, flat panel displays, backlights, illumination, interior decoration, signs, signboards, electron cameras and optical signal generators.

## Claims

1. A light emitting device material used for a light emitting device, which includes at least an emissive layer having a host and a dopant and is present between an anode and a cathode and emits light by electrical energy, wherein the light emitting device material is a light emitting device material for a dopant, having a dibenzochrysene skeleton represented by the following general formula (1): 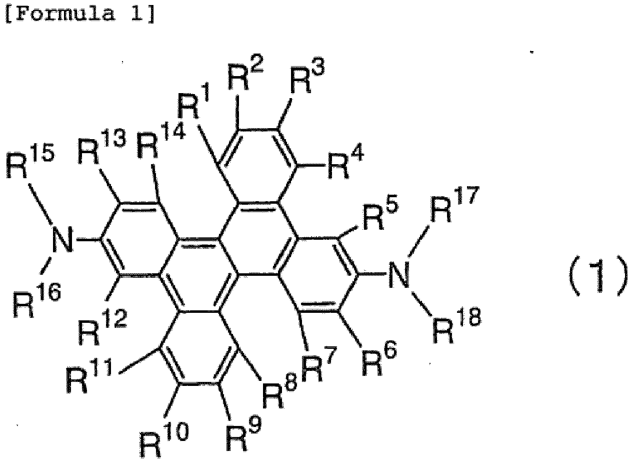 wherein each of R¹ to R¹⁴ is selected from the group consisting of hydrogen, an alkyl group, a cycloalkyl group, an aryl group, a heterocyclic group, a heteroaryl group, an alkenyl group, a cycloalkenyl group, an alkynyl group, an alkoxy group, an alkylthio group, an aryl ether group, an arylthio ether group, a halogen atom, a cyano group, - P(=O)R¹⁹R²⁰ and a silyl group, any two substituents adjacent to each other of R¹ to R¹⁴ may form a ring, R¹⁹ and R²⁰ are respectively an aryl group or a heteroaryl group, R¹⁵ to R¹⁸ may be the same or different and each of them is selected from the group consisting of an alkyl group, a cycloalkyl group, an aryl group, a heterocyclic group and a heteroaryl group and when each of R¹⁵ to R¹⁸ is an aryl group or a heterocyclic group, R¹⁵ and R¹⁶ or R¹⁷ and R¹⁸ may be coupled with each other to form a saturated or an unsaturated ring.

2. The light emitting device material according to claim 1, wherein at least one of R¹⁵ to R¹⁸ is represented by the following general formula (2): 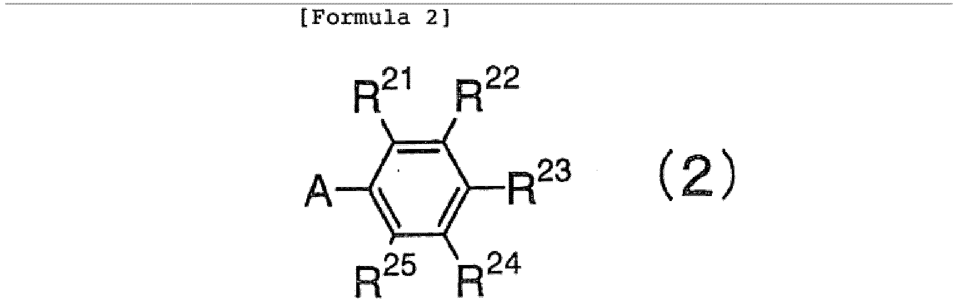 wherein A is used for coupling with a nitrogen atom, each of R²¹ to R²⁵ is selected from the group consisting of hydrogen, an aryl group, an alkyl group, a cycloalkyl group, an alkoxy group, an alkylthio group, an aryl ether group, an arylthio ether group, a silyl group and a fused ring formed between adjacent substituents, provided that at least one of R²¹ to R²⁵ is an aryl group, an alkyl group or a cycloalkyl group, or at least one pair of groups adjacent to each other of R²¹ to R²⁵ is a fused ring formed between adjacent substituents.

3. The light emitting device material according to claim 2, wherein at least one of R²¹ to R²⁵ is an alkyl group or a cycloalkyl group.

4. The light emitting device material according to claim 1, wherein R¹⁵ to R¹⁸ is an aryl group, and wherein at least one of R¹⁵ to R¹⁸ is represented by the following general formula (4): 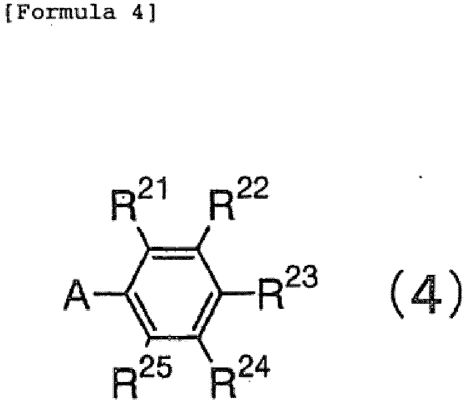 wherein A is used for coupling with a nitrogen atom, each of R²¹ to R²⁵ is selected from the group consisting of hydrogen, an aryl group, an alkyl group, a cycloalkyl group, an alkoxy group, an alkylthio group, an aryl ether group, an arylthio ether group, a silyl group and a fused ring formed between adjacent substituents, provided that at least one of R²¹ to R²⁵ is an aryl group, an alkyl group or a cycloalkyl group, or at least one pair of groups adjacent to each other of R²¹ to R²⁵ is a fused ring formed between adjacent substituents.

5. Use of the light emitting device material according to claim 4 as a hole injection material in a light emitting device including at least a hole injection layer and an emissive layer present between an anode and a cathode and emitting light by electrical energy.

6. A light emitting device including at least a hole injection layer and an emissive layer present between an anode and a cathode and emitting light by electrical energy, wherein the light emitting device material according to claim 4 is contained in the hole injection layer.

7. A light emitting device including at least an emissive layer present between an anode and a cathode and emitting light by electrical energy, wherein the emissive layer has a host and a dopant and contains the light emitting device material according to any one of claims 1 to 3 as the dopant.

8. The light emitting device according to claim 7, wherein the host is selected from an anthracene compound and a pyrene compound.

9. The light emitting device according to any of claims 6 to 8, wherein at least an electron transporting layer is present between the emissive layer and the cathode, and the electron transporting layer includes electron-accepting nitrogen and contains a compound having a heteroaryl ring structure composed of elements selected from carbon, hydrogen, nitrogen, oxygen, silicon, and phosphorus.

## Patentansprüche

1. Lichtemittierendes Vorrichtungsmaterial, welches für eine lichtemittierende Vorrichtung verwendet wird, welches mindestens eine emissive Schicht mit einem Wirtsmaterial und einem Dotiermaterial aufweist und zwischen einer Anode und einer Kathode vorhanden ist und Licht durch elektrische Energie emittiert, wobei das lichtemittierende Vorrichtungsmaterial ein lichtemittierendes Vorrichtungsmaterial für ein Dotiermaterial mit einem Dibenzochrysengerüst, welches durch die allgemeine Formel (1) dargestellt ist, ist: 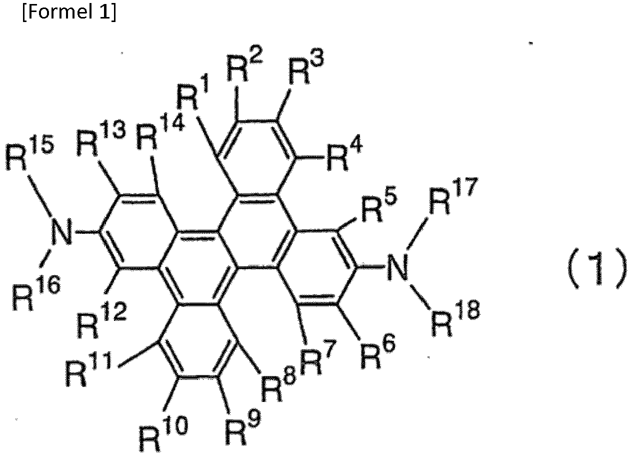 wobei jeder R¹ bis R¹⁴ ausgewählt ist aus der Gruppe bestehend aus Wasserstoff, einer Alkylgruppe, einer Cycloalkylgruppe, einer Arylgruppe, einer heterocyclischen Gruppe, einer Heteroarylgruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einem Halogenatom, einer Cyanogruppe, -P(=O)R¹⁹R²⁰ und einer Silylgruppe, wobei beliebige zwei Substituenten, welche benachbart zueinander sind und aus R¹ bis R¹⁴ einen Ring bilden können, R¹⁹ und R²⁰ jeweils eine Arylgruppe oder eine Heteroarylgruppe sind, R¹⁵ bis R¹⁸ gleich oder verschieden sein können und jeder davon ausgewählt ist aus der Gruppe bestehend aus einer Alkylgruppe, einer Cycloalkylgruppe, einer Arylgruppe, einer heterocyclischen Gruppe und einer Heteroarylgruppe und, wenn jedes R¹⁵ bis R¹⁸ eine Arylgruppe oder eine heterocyclische Gruppe ist, R¹⁵ und R¹⁶ oder R¹⁷ und R¹⁸ aneinander gekuppelt werden können, um einen gesättigten oder einen ungesättigten Ring zu bilden.

2. Lichtemittierendes Vorrichtungsmaterial nach Anspruch 1, wobei mindestens einer von R¹⁵ bis R¹⁸ durch die folgende allgemeine Formel (2) dargestellt ist: 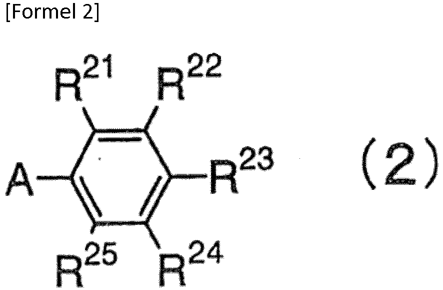 wobei A verwendet wird, um mit einem Stickstoffatom, jedem von R²¹ bis R²⁵, ausgewählt aus der Gruppe bestehend aus Wasserstoff, einer Arylgruppe, einer Alkylgruppe, einer Cycloalkylgruppe, einer Alkoxygruppe, einer Alkylthiogruppe, einer Arylethergruppe, einer Arylthioethergruppe, einer Silylgruppe und einem gebildeten Ring ist, welcher zwischen benachbarten Substituenten, sofern mindestens einer aus R²¹ bis R²⁵ eine Arylgruppe, eine Alkylgruppe oder eine Cycloalkylgruppe ist, oder zumindest ein paar von Gruppen, welches benachbart zu R²¹ bis R²⁵ ist, ein gebildeter Ring, welcher zwischen benachbarten Substituenten gebildet wird, zu kuppeln.

3. Lichtemittierendes Vorrichtungsmaterial nach Anspruch 2, wobei mindestens einer von R²¹ bis R²⁵ eine Alkylgruppe oder eine Cycloalkylgruppe ist.

4. Lichtemittierendes Vorrichtungsmaterial nach Anspruch 1, wobei R¹⁵ bis R¹⁸ eine Arylgruppe ist, und wobei mindestens einer von R¹⁵ bis R¹⁸ durch die folgende allgemeine Formel (4) dargestellt ist: 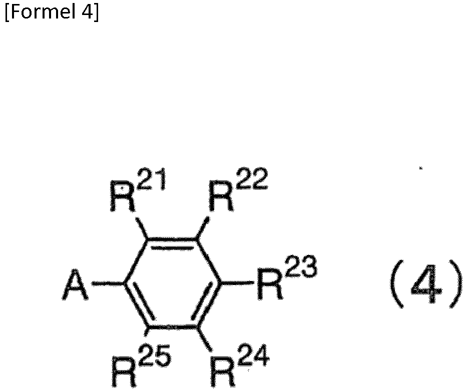 wobei A verwendet wird, um mit einem Stickstoffatom, jedem von R²¹ bis R²⁵, ausgewählt aus der Gruppe bestehend aus Wasserstoff, einer Arylgruppe, einer Alkylgruppe, einer Cycloalkylgruppe, einer Alkoxygruppe, einer Alkylthiogruppe, einer Arylethergruppe, einer Arylthioethergruppe, einer Silylgruppe und einem gebildeten Ring, welcher zwischen benachbarten Substituenten, sofern mindestens einer aus R²¹ bis R²⁵ eine Arylgruppe, eine Alkylgruppe oder eine Cycloalkylgruppe ist, oder zumindest ein paar von Gruppen, welches benachbart zu R²¹ bis R²⁵ ist, ein gebildeter Ring ist, welcher zwischen benachbarten Substituenten gebildet wird, zu kuppeln.

5. Verwendung des lichtemittierenden Vorrichtungsmaterials nach Anspruch 4 als ein Löcherinjektionsmaterial in einer lichtemittierenden Vorrichtung umfassend mindestens eine Löcherinjektionsschicht und eine emissive Schicht, welche zwischen einer Anode und einer Kathode vorhanden ist und Licht durch elektrische Anregung emittiert.

6. Lichtemittierende Vorrichtung umfassend mindestens eine Löcherinjektionsschicht und eine emissive Schicht, welche zwischen einer Anode und einer Kathode vorhanden ist und Licht durch elektrische Energie emittiert, wobei das lichtemittierende Vorrichtungsmaterial nach Anspruch 4 in der Löcherinjektionsschicht enthalten ist.

7. Lichtemittierende Vorrichtung umfassend mindestens eine emissive Schicht, welche zwischen der Anode und der Kathode vorhanden ist und Licht durch elektrische Energie emittiert, wobei die emissive Schicht ein Wirtsmaterial und ein Dotiermaterial aufweist und das lichtemittierende Vorrichtungsmaterial nach einem der Ansprüche 1 bis 3 als das Dotierungsmittel enthält.

8. Lichtemittierende Vorrichtung nach Anspruch 7, wobei das Wirtsmaterial ausgewählt ist aus einer Anthracenverbindung und einer Pyrenverbindung.

9. Lichtemittierende Vorrichtung nach einem der Ansprüche 6 bis 8, wobei mindestens eine Elektronentransportschicht zwischen der emissiven Schicht und der Kathode vorhanden ist, und die Elektronentransportschicht elektronenakzeptierenden Stickstoff enthält und eine Verbindung mit einer Heteroarylringstruktur, welche sich aus Elementen ausgewählt aus Kohlenstoff, Wasserstoff, Stickstoff, Sauerstoff, Silicium und Phosphor zusammensetzt, enthält.

## Revendications

1. Matériau de dispositif électroluminescent utilisé pour un dispositif électroluminescent, qui comprend au moins une couche émissive ayant un hôte et un dopant et est présente entre une anode et une cathode et émet une lumière par une énergie électrique, dans lequel le matériau de dispositif électroluminescent est un matériau de dispositif électroluminescent pour un dopant, possédant un squelette de dibenzochrysène représenté par la formule générale (1) suivante : 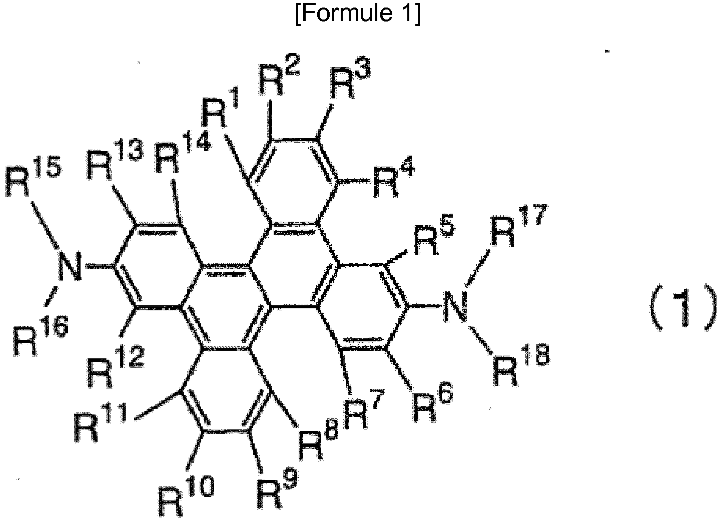 dans laquelle chacun parmi R¹ à R¹⁴ est sélectionné dans le groupe constitué d'un hydrogène, d'un groupe alkyle, d'un groupe cycloalkyle, d'un groupe aryle, d'un groupe hétérocyclique, d'un groupe hétéroaryle, d'un groupe alcényle, d'un groupe cycloalcényle, d'un groupe alcynyle, d'un groupe alcoxy, d'un groupe alkylthio, d'un groupe aryl éther, d'un groupe arylthio éther, d'un atome d'halogène, d'un groupe cyano, de -P(=O)R¹⁹R²⁰ et d'un groupe silyle, deux substituants quelconques adjacents l'un à l'autre parmi R¹ à R¹⁴ peuvent former un cycle, R¹⁹ et R²⁰ sont respectivement un groupe aryle ou un groupe hétéroaryle, R¹⁵ à R¹⁸ peuvent être identiques ou différents et chacun d'eux est sélectionné dans le groupe constitué d'un groupe alkyle, d'un groupe cycloalkyle, d'un groupe aryle, d'un groupe hétérocyclique et d'un groupe hétéroaryle et quand chacun parmi R¹⁵ à R¹⁸ est un groupe aryle ou un groupe hétérocyclique, R¹⁵ et R¹⁶ ou R¹⁷ et R¹⁸ peuvent être couplés l'un à l'autre pour former un cycle saturé ou insaturé.

2. Matériau de dispositif électroluminescent selon la revendication 1, dans lequel au moins l'un parmi R¹⁵ à R¹⁸ est représenté par la formule générale (2) suivante : 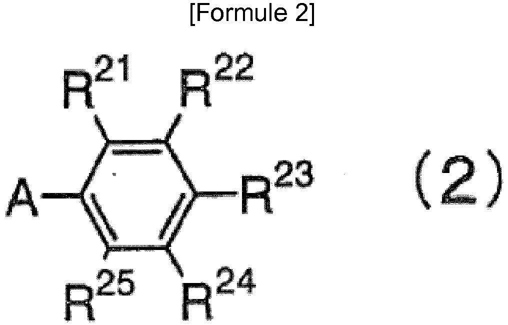 dans laquelle A est utilisé pour un couplage avec un atome d'azote, chacun parmi R²¹ à R²⁵ est sélectionné dans le groupe constitué d'un hydrogène, d'un groupe aryle, d'un groupe alkyle, d'un groupe cycloalkyle, d'un groupe alcoxy, d'un groupe alkylthio, d'un groupe aryl éther, d'un groupe arylthio éther, d'un groupe silyle et d'un cycle fusionné formé entre des substituants adjacents, à condition qu'au moins l'un parmi R²¹ à R²⁵ soit un groupe aryle, un groupe alkyle ou un groupe cycloalkyle, ou qu'au moins une paire de groupes adjacents l'un à l'autre parmi R²¹ à R²⁵ soit un cycle fusionné formé entre des substituants adjacents.

3. Matériau de dispositif électroluminescent selon la revendication 2, dans lequel au moins l'un parmi R²¹ à R²⁵ est un groupe alkyle ou un groupe cycloalkyle.

4. Matériau de dispositif électroluminescent selon la revendication 1, dans lequel R¹⁵ à R¹⁸ est un groupe aryle, et dans lequel au moins l'un parmi R¹⁵ à R¹⁸ est représenté par la formule générale (4) suivante : 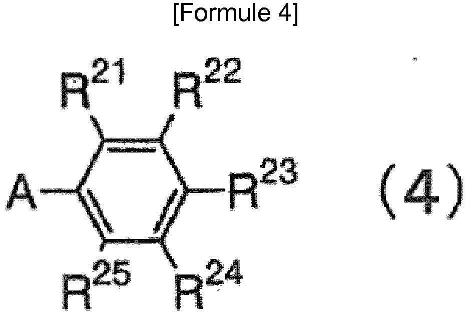 dans laquelle A est utilisée pour un couplage avec un atome d'azote, chacun parmi R²¹ à R²⁵ est sélectionné dans le groupe constitué d'un hydrogène, d'un groupe aryle, d'un groupe alkyle, d'un groupe cycloalkyle, d'un groupe alcoxy, d'un groupe alkylthio, d'un groupe aryl éther, d'un groupe arylthio éther, d'un groupe silyle et d'un cycle fusionné formé entre des substituants adjacents, à condition qu'au moins l'un parmi R²¹ à R²⁵ soit un groupe aryle, un groupe alkyle ou un groupe cycloalkyle, ou qu'au moins une paire de groupes adjacents l'un à l'autre parmi R²¹ à R²⁵ soit un cycle fusionné formé entre des substituants adjacents.

5. Utilisation du matériau de dispositif électroluminescent selon la revendication 4 comme matériau d'injection de trous dans un dispositif électroluminescent comprenant au moins une couche d'injection de trous et une couche émissive présentes entre une anode et une cathode et émettant une lumière par une énergie électrique.

6. Dispositif électroluminescent comprenant au moins une couche d'injection de trous et une couche émissive présentes entre une anode et une cathode et émettant une lumière par une énergie électrique, dans lequel le matériau de dispositif électroluminescent selon la revendication 4 est contenu dans la couche d'injection de trous.

7. Dispositif électroluminescent comprenant au moins une couche émissive présente entre une anode et une cathode et émettant une lumière par une énergie électrique, dans lequel la couche émissive a un hôte et un dopant et contient le matériau de dispositif électroluminescent selon l'une quelconque des revendications 1 à 3 en tant que dopant.

8. Dispositif électroluminescent selon la revendication 7, dans lequel l'hôte est sélectionné parmi un composé d'anthacène et un composé de pyrène.

9. Dispositif électroluminescent selon l'une quelconque des revendications 6 à 8, dans lequel au moins une couche de transport d'électrons est présente entre la couche émissive et la cathode, et la couche de transport d'électrons comprend un azote accepteur d'électrons et contient un composé ayant une structure de cycle hétéroaryle composée d'éléments sélectionnés parmi le carbone, l'hydrogène, l'azote, l'oxygène, le silicium, et le phosphore.
